# EUROPEAN PATENT APPLICATION

(11) **EP 2 439 437 A1**
(43) Date of publication of application: **11.04.2012**
(21) Application number: 10783469.9
(22) Date of filing: 04.06.2010
(51) Int. Cl.: F16L 19/02, A61M 39/02

(54) **FEMALE CONNECTOR, MALE CONNECTOR, CONNECTION STRUCTURE, LIQUID SUPPLY CIRCUIT, AND MEDICAL CONTAINER**

(30) Priority: 04.06.2009 JP 2009135379; 06.05.2010 JP 2010106771
(71) Applicant: JMS. Co., Ltd., Hiroshima 730-8652 (US)
(72) Inventor: KUNISHIGE, Takahiko, Hiroshima-shi, Hiroshima 730-8652 (JP); KOBASHI, Yoshihiko, Hiroshima-shi, Hiroshima 730-8652 (JP)
(74) Representative: Stippl, Hubert
(86) International application number: PCT/JP2010/059556
(87) International publication number: WO 2010/140687

(57) **Abstract**

In a female connector 15 of the present invention, a surface of an engagement wall 217 facing an engagement tab 14 when the female connector 15 is connected to a male connector 10 includes an inclined surface 222 which the engagement tab 14 mounts in a sliding direction of the engagement tab 14 with respect to the engagement wall 217 during the connection between the female connector 15 and the male connector 10. Further, in a male connector 30 of the present invention, a surface of the engagement tab 34 opposed to the engagement wall 417 when the female connector 35 is connected to the male connector 30 includes an inclined surface 34a which the engagement wall 417 mounts in a sliding direction of the engagement wall 417 with respect to the engagement tab 34 when during the connection between the female connector 35 and the male connector 30 .

## Description

### Technical Field

The present invention relates to a female connector, a male connector, a connection structure, a liquid-sending circuit including the female connector, and a medical container including the male connector.

### Background Art

As a method for administering a nutrient or a drug to a patient parenterally, transintestine nutrition therapy and intravenous nutrition therapy are known. According to the transintestine nutrition therapy, a liquid-like substance such as a nutrient, a liquid diet, or a drug (generally called a "transintestine nutrient") is administered through a tube running from the nasal cavity to the stomach or the duodenum of a patient (generally called a "nasotracheal tube") or through a tube inserted in a gastric fistula formed in the abdomen of the patient (generally called a "PEG tube") (the operation for forming a gastric fistula is called "Percutaneous Endoscopic Gastrostomy"). Further, according to the intravenous nutrition therapy, a liquid-like substance containing a nutrient component such as glucose or a drug component (generally called an "infusion solution") is administered through an infusion solution line inserted in the vein of a patient.

The transintestine nutrition therapy through a nasotracheal tube will be described. A liquid-like substance to be administered to a patient is stored in a medical container. A male connector, out of which the liquid-like substance flows, is provided at a lower end of the medical container. The liquid-like substance in the medical container generally is administered to a patient through a transintestine nutrient supply set and a nasotracheal tube. A female connector to be connected to the male connector is provided at one end of a tube constituting the transintestine nutrient supply set (see, for example, Patent Document 1).

As shown in FIG. 32, the female connector described in Patent Document 1 is a female connector 1000 to be connected to a male connector 900 having a tubular section 972, out of which a liquid-like substance flows, and the female connector 1000 includes an adaptor 1010 installed on an outer circumferential surface of the tubular section 972 and a handle 1020 fitted on the adaptor 1010. The adaptor 1010 has a tube-shaped section 1012 having an opening at one end thereof, the tube-shaped section 1012 includes an insertion section 1019 into which the tubular section 972 is inserted from the opening, and the insertion section 1019 has flexibility and elasticity. The handle 1020 is more rigid than the insertion section 1019. The connection of the female connector 1000 to the male connector 900 is performed as follows.

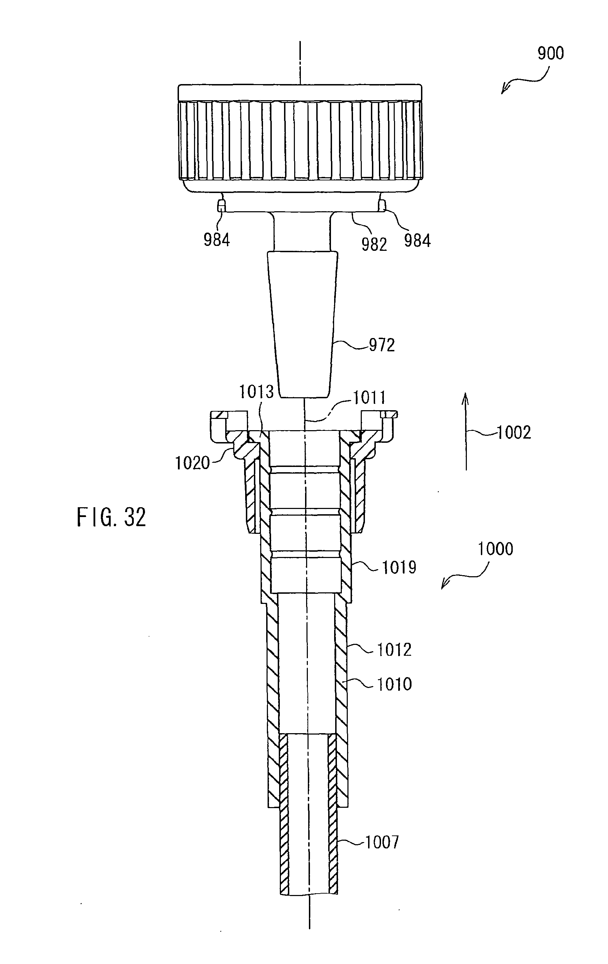

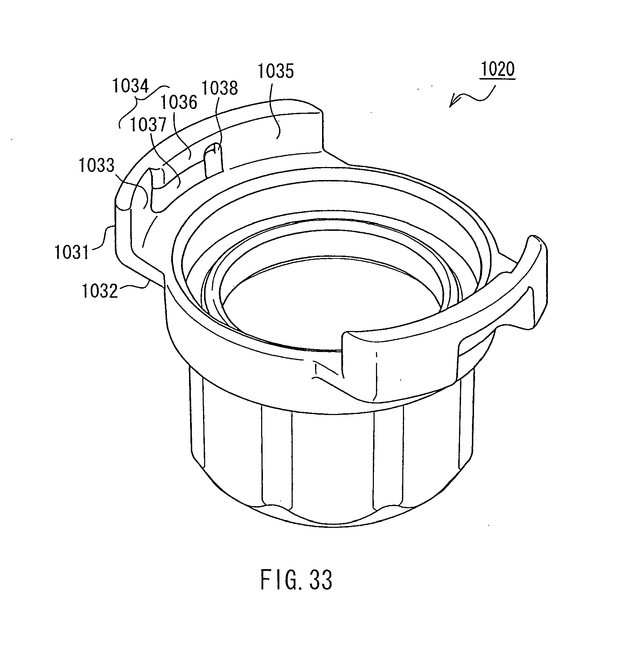

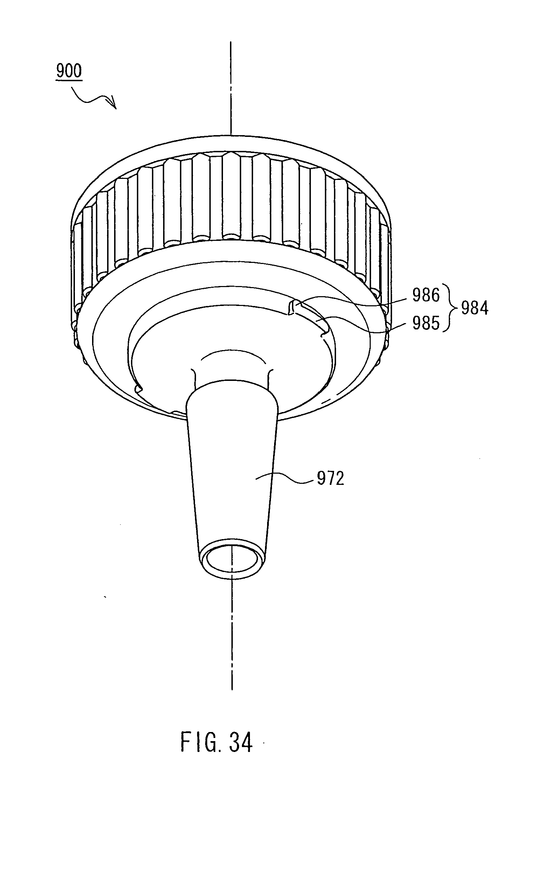

First, as shown in FIG. 32, after the tubular section 972 of the male connector 900 is inserted deeply into the insertion section 1019 of the adaptor 1010, the handle 1020 further is pushed in a direction indicated by an arrow 1002 with respect to the male connector 900, and thus, the upper surfaces of bridge sections 1032 (see FIG. 33) of the handle 1020 abut on the lower surface of a platform 982 of the male connector 900. In this state, the handle 1020 is rotated clockwise around a center axis 1011 with respect to the male connector 900. A pair of engagement tabs 984 formed on the outer circumferential surface of the platform 982 of the male connector 900 pass through a pair of passage regions 1033 of arc-shaped walls 1031 of the handle 1020 to reach engagement regions 1034 and are stored in concave portions 1037. After the engagement tabs 984 of the male connector 900 abut on ends of no-passage regions 1035 on the sides of the engagement regions 1034, the handle 1020 cannot be rotated any more with respect to the male connector 900. When the hand is taken off the handle 1020 in this state, the handle 1020 moves slightly in the direction opposite to the arrow 1002 due to the elastic recovery force of a flange section 1013 of the adaptor 1010 that is deformed elastically, lock protrusions 986 (see FIG. 34) of the engagement tabs 984 of the male connector 900 are fitted in lock concave portions 1038 of the handle 1020, and engagement chips 985 of the engagement tabs 984 of the male connector 900 and engagement walls 1036 of the handle 1020 are engaged with each other.

### Prior Art Document

### Patent Document

Patent document 1: WO 2008/52871

### Disclosure of Invention

### Problem to be Solved by the Invention

However, the adaptor 1010 is made of, for example, a material having flexibility and elasticity such as natural rubber, or synthetic rubber, and the handle 1020 is made of a material (for example, polypropylene resin) having a hardness higher than that of the adaptor 1010, and the adaptor 1010 and the handle 1020 are formed separately. Therefore, for producing the female connector described in Patent Document 1, there is a problem that two kinds materials and assembly steps are required, which increases cost.

Further, since the adaptor 1010 is made of a material having flexibility and elasticity, it is difficult to perform a connection operation of inserting a liquid-sending tube 1007 having flexibility into the adaptor 1010.

In order to solve these problems, it is considered that an adaptor and a handle are formed by so-called integral molding in which the adaptor and the handle are molded simultaneously and integrated in a common mold, using a material (for example, polypropylene resin) used as a material for the handle in Patent Document 1. However, in this case, the insertion resistance of the tubular section 972 of the male connector 900 with respect to the insertion section 1019 of the adaptor 1010 increases. Further, if the tubular section 972 cannot be inserted into the insertion section 1019 sufficiently, it becomes difficult to perform the rotation operation to be conducted so as to allow the engagement tabs 984 to pass through the passage regions 1033 shown in FIG. 33 to reach the engagement regions 1034, since the sliding resistance of the engagement tabs 984 with respect to the arc-shaped walls 1031 becomes extremely large.

The insertion resistance and sliding resistance can be decreased, for example, by enlarging the inner diameter of the insertion section 1019 or gradually enlarging the inner diameter of the insertion section 1019 in the direction indicated by the arrow 1002 to reduce the sliding area of the tubular section 972 with respect to the insertion section 1019 so as to facilitate the insertion of the tubular section 972 into the insertion section 1019. However, in this case, there is a problem that the liquid-tightness to be maintained by the contact between the outer circumferential surface of the tubular section 972 and the inner circumferential surface of the insertion section 1019 is impaired by enhancing the connection operability between the female connector and the male connector.

Accordingly, the present invention provides a female connector, a male connector, a connection structure, a liquid-sending circuit including the female connector, and a medical container including the male connector, which are capable of reducing cost, enhancing the connection operability between the female connector and the male connector, and enhancing the connection operability of a liquid-sending tube with respect to the female connector without impairing the above-mentioned liquid-tightness.

### Means for Solving Problem

A female connector of the present invention is a female connector to be connected to a male connector having a tubular section through which a liquid-like substance flows out,
wherein the male connector includes:
a platform in which the tubular section is provided so as to protrude from a center of one principal surface; and
an engagement tab protruding outward from an outer circumferential surface of the platform,
the female connector includes:
   a tube-shaped section to be connected to the tubular section;
   a bottom section connected to one end of the tube-shaped section;
   a liquid-sending tube connecting section to which a liquid-sending tube is to be connected, one end of the liquid-sending tube connecting section being connected to the tube-shaped section through the bottom section of the female connector, and an inside of the liquid-sending tube connecting section being communicated with an inside of the tubular section when the female connector is connected to the male connector; and
   a brim section provided so that at least a part thereof protrudes to a side of the tube-shaped section opposite to the liquid-sending tube connecting section side,
   an inner circumferential surface of the brim section opposed to a center axis of the tube-shaped section includes an engagement wall protruding inward with a longitudinal direction thereof being in a circumferential direction, and a part of the inner circumferential surface of the brim section on the tube-shaped section side relative to the engagement wall, which is recessed with respect to the center axis relative to the engagement wall due to the presence of the engagement wall, constitutes a concave portion in which the engagement tab is to be stored, and
   a surface of the engagement wall facing the engagement tab when the female connector is connected to the male connector includes an inclined surface that the engagement tab mounts in a sliding direction of the engagement tab with respect to the engagement wall during the connection between the female connector and the male connector.

A male connector of the present invention is a male connector to be connected to a female connector, having a tubular section through which a liquid-like substance flows out,
wherein the male connector includes:
a platform in which the tubular section is provided so as to protrude from a center of one principal surface; and
an engagement tab protruding outward from an outer circumferential surface of the platform,
the female connector includes:
   a tube-shaped section to be connected to the tubular section;
   a bottom section connected to one end of the tube-shaped section;
   a liquid-sending tube connecting section to which a liquid-sending tube is to be connected, one end of the liquid-sending tube connecting section being connected to the tube-shaped section through the bottom section of the female connector, and an inside of the liquid-sending tube connecting section being communicated with an inside of the tubular section when the female connector is connected to the male connector; and
   a brim section provided so that at least a part thereof protrudes to a side of the tube-shaped section opposite to the liquid-sending tube connecting section side,
   an inner circumferential surface of the brim section opposed to a center axis of the tube-shaped section includes an engagement wall protruding inward with a longitudinal direction thereof being in a circumferential direction, and a part of the inner circumferential surface of the brim section on the tube-shaped section side relative to the engagement wall, which is recessed with respect to the center axis relative to the engagement wall due to the presence of the engagement wall, constitutes a concave portion in which the engagement tab is to be stored, and
   a surface of the engagement tab facing the engagement wall when the female connector is connected to the male connector includes an inclined surface that the engagement wall mounts in a sliding direction of the engagement wall with respect to the engagement tab during the connection between the female connector and the male connector.

A first connection structure of the present invention is a connection structure of the above-mentioned female connector of the present invention and a male connector having a tubular section through which a liquid-like substance flows out,
wherein the male connector includes:
a platform in which the tubular section is provided so as to protrude from a center of one principal surface; and
an engagement tab protruding outward from an outer circumferential surface of the platform, and
the engagement tab and the engagement wall of the female connector are engaged with each other when the engagement tab is stored in the concave portion of the female connector.

A second connection structure of the present invention is a connection structure of the above-mentioned male connector of the present invention and a female connector, wherein the female connector includes:
a tube-shaped section to be connected to the tubular section;
a bottom section connected to one end of the tube-shaped section;
a liquid-sending tube connecting section to which a liquid-sending tube is to be connected, one end of the liquid-sending tube connecting section being connected to the tube-shaped section through the bottom section of the female connector, and an inside of the liquid-sending tube connecting section being communicated with an inside of the tubular section when the female connector is connected to the male connector; and
a brim section provided so that at least a part thereof protrudes to a side of the tube-shaped section opposite to the liquid-sending tube connecting section side,
an inner circumferential surface of the brim section opposed to a center axis of the tube-shaped section includes an engagement wall protruding inward with a longitudinal direction thereof being in a circumferential direction, and a part of the inner circumferential surface of the brim section on the tube-shaped section side relative to the engagement wall, which is recessed with respect to the center axis relative to the engagement wall due to the presence of the engagement wall, constitutes a concave portion in which the engagement tab is to be stored, and
the engagement tab and the engagement wall are engaged with each other when the engagement tab of the male connector is stored in the concave portion.

Another male connector of the present invention is a male connector to be connected to a female connector, having a tubular section through which a liquid-like substance flows out,
wherein the male connector includes:
a platform in which the tubular section is provided so as to protrude from a center of one principal surface; and
a brim section that protrudes from the one principal surface of the platform to the tubular section side and is placed around the tubular section,
the female connector includes:
   an insertion section in which the tubular section is to be inserted;
   a liquid-sending tube connecting section to which a liquid-sending tube is to be connected, the liquid-sending tube connecting section being connected to the insertion section and an inside of the liquid-sending tube connecting section being communicated with an inside of the tubular section when the female connector is connected to the male connector;
   a flange section opposed to the platform when the female connector is connected to the male connector; and
   an engagement tab protruding outward from an outer circumferential surface of the flange section,
   an inner circumferential surface of the brim section opposed to a center axis of the tubular section of the male connector includes an engagement wall protruding inward with a longitudinal direction thereof being in a circumferential direction, and a part of the inner circumferential surface of the brim section on the platform side relative to the engagement wall, which is recessed with respect to the center axis relative to the engagement wall due to the presence of the engagement wall, constitutes a storage section in which the engagement tab is to be stored, and
   a surface of the engagement wall facing the engagement tab when the female connector is connected to the male connector includes an inclined surface that the engagement tab mounts in a sliding direction of the engagement tab with respect to the engagement wall during the connection between the female connector and the male connector.

A liquid-sending circuit of the present invention includes: the female connector of the present invention; and a liquid-sending tube connected to the liquid-sending tube connecting section of the female connector.

A medical container of the present invention includes:
a container main section having a hollow cylindrical port for injecting a liquid-like substance into the container main section or taking out the liquid-like substance therefrom; and
the male connector of the present invention,
wherein the male connector is integrated with the port in the course of molding or connected to the port removably through connection means.

In the present application, the male connector to be connected to an example of the female connector of the present invention includes those which are not the male connector according to the present invention, and hereinafter, such a male connector will be referred to as a "male connector Q" so as to be discriminated from the male connector of the present invention. The female connector of the present invention to be connected to the "male connector Q" will be, hereinafter, referred to as a "female connector A". A connection structure of the male connector Q and the female connector A is the above-mentioned first connection structure.

The present application also discloses a male connector C of the present invention to be connected to the female connector A of the present invention.

The female connector to be connected to a male connector P of the present invention will be, hereinafter, referred to as a "female connector B" so as to be discriminated from the "female connectorA" to be connected to the above-mentioned "male connector Q". The male connector of the present invention to be connected to the "female connector B will be, hereinafter, referred to as the "male connector P" so as to be discriminated from the "male connector Q". The connection structure of the male connector P and the female connector B is the above-mentioned second connection structure.

The present application also discloses a male connector E of the present invention different from the male connector Q and the male connector P of the present invention, and the female connector to be connected to the male connector E will be referred to as a "female connector D" so as to be discriminated from the other female connectors.

### Effects of the Invention

In the female connector A of the present invention, a surface of an engagement wall facing an engagement tab when the female connector A is connected to the male connector Q or the male connector C includes an inclined surface with a gradient, which the engagement tab mounts in a sliding direction of the engagement tab with respect to the engagement wall during the connection between the female connector A and the male connector Q or the male connector C. Further, in the male connector P of the present invention, a surface of an engagement tab facing an engagement wall when the female connector B is connected to the male connector P includes an inclined surface with a gradient, which the engagement wall mounts in a sliding direction of the engagement wall with respect to the engagement tab during the connection between the female connector B and the male connector P. Further, in the male connector E of the present invention, a surface of an engagement wall of the male connector E, facing an engagement tab of the female connector D when the female connector D is connected to the male connector E includes an inclined surface that the engagement tab mounts in a sliding direction of the engagement tab with respect to the engagement wall during the connection between female connector D and the male connector E.

Therefore, when a tubular section is inserted in a deeper portion of a tube-shaped section, or when the tube-shaped section is inserted in a deeper portion of the tubular section, the tubular section and the tube-shaped section can be connected to each other easily, and liquid-tightness also can be ensured. Further, since the tubular section and the tube-shaped section are connected to each other easily, the sliding resistance of the engagement tab with respect to the engagement wall also can be decreased.

Further, the female connector A of the present invention, the female connector B, and the female connector D are made of materials having a hardness higher than that of a material having flexibility and elasticity such as natural rubber, or synthetic rubber. Therefore, a liquid-sending tube can be connected to a liquid-sending tube connecting section easily, and in particular, the liquid-sending tube can be easily connected to the liquid-sending tube connecting section by a machine. Thus, a cost also can be reduced.

### Brief Description of Drawings

[FIG. 1A] FIG. 1A is a front view showing an example of a schematic configuration of a medical container having an example of a male connector Q (male connector 10) to which an example of a female connector A (female connector 15) of the present invention is to be connected.
[FIG. 1B] FIG. 1B is a bottom view of the medical container shown in FIG. 1A.
[FIG. 2A] FIG. 2A is a partially enlarged perspective view of the medical container of FIG. 1A.
[FIG. 2B] FIG. 2B is a partially enlarged front view of the medical container of FIG. 1A
[FIG. 2C] FIG. 2C is a partially enlarged bottom view of the medical container of FIG. 1A (it should be noted that a container main section is omitted).
[FIG. 3A] FIG. 3A is a perspective view showing a schematic configuration of the female connector 15.
[FIG. 3B] FIG. 3B is a plan view of the female connector 15 shown in FIG. 3A.
[FIG. 3C] FIG. 3C is a front view of the female connector 15 shown in FIG. 3A.
[FIG. 3D] FIG. 3D is a cross-sectional view taken along a line A-A' of the female connector 15 shown in FIG. 3A.
[FIG. 3E] FIG. 3E is a cross-sectional view taken along a line B-B' of the female connector 15 shown in FIG. 3A.
[FIG. 4] FIG. 4 is a cross-sectional view illustrating a state of the connection between the male connector 10 shown in FIG. 2A and the female connector 15 shown in FIG. 3A.
[FIG. 5A] FIG. 5A is a perspective view illustrating a state of the connection between the male connector 10 shown in FIG. 2A and the female connector 15 shown in FIG. 3A.
[FIG. 5B] FIG. 5B is a perspective view illustrating a state of the connection between the male connector 10 shown in FIG. 2A and the female connector 15 shown in FIG. 3A.
[FIG. 6] FIG. 6 is a cross-sectional view illustrating a state in which the male connector 10 shown in FIG. 2A and the female connector 15 shown in FIG. 3A are connected to each other.
[FIG. 7A] FIG. 7A is a front view showing an example of a schematic configuration of a medical container including an example of a male connector P (male connector 30) of the present invention to which an example of a female connector B (female connector 35) is to be connected.
[FIG. 7B] FIG. 7B is a bottom view of the medical container shown in FIG. 7A.
[FIG. 8A] FIG. 8A is a partially enlarged perspective view of the medical container of FIG. 7A.
[FIG. 8B] FIG. 8B is a partially enlarged front view of the medical container of FIG. 7A.
[FIG. 8C] FIG. 8C is a partially enlarged bottom view of the medical container of FIG. 7A (it should be noted that a container main section is omitted).
[FIG. 9A] FIG. 9A is a perspective view showing a schematic configuration of the female connector 35 to be connected to the male connector 30 shown in FIG. 7A.
[FIG. 9B] FIG. 9B is a plan view of the female connector 35 shown in FIG. 9A.
[FIG. 9C] FIG. 9C is a front view of the female connector 35 shown in FIG. 9A.
[FIG. 9D] FIG. 9D is a cross-sectional view taken along a line A-A' of the female connector 35 shown in FIG. 9A.
[FIG. 9E] FIG. 9E is a cross-sectional view taken along a line B-B' of the female connector 35 shown in FIG. 9A.
[FIG. 10] FIG. 10 is a cross-sectional view illustrating a state of the connection between the male connector 30 shown in FIG. 8A and the female connector 35 shown in FIG. 9A.
[FIG. 11A] FIG. 11A is a perspective view illustrating a state of the connection between the male connector 30 shown in FIG. 8A and the female connector 35 shown in FIG. 9A.
[FIG. 11B] FIG. 11B is a perspective view illustrating a state of the connection between the male connector 30 shown in FIG. 8A and the female connector 35 shown in FIG. 9A.
[FIG. 12] FIG. 12 is a cross-sectional view illustrating a state in which the male connector 30 shown in FIG. 8A and the female connector 35 shown in FIG. 9A are connected to each other.
[FIG. 13] FIG. 13 is an exploded view showing a schematic configuration of a medical container including another example of the male connector P (male connector 60) of the present invention.
[FIG. 14] FIG. 14 is a front view showing an example of a schematic configuration of a transintestine nutrient supply set that includes a liquid-sending circuit including the female connector 15 or the female connector 35.
[FIG. 15] FIG. 15 is a front view showing an example of a schematic configuration of a medical container including another example of the male connector P (male connector 70) of the present invention.
[FIG. 16] FIG. 16 is a cross-sectional view showing a state in which a cap is attached to the male connector 30 shown in FIGS. 8A and 8B.
[FIG. 17A] FIG. 17A is a perspective view showing a schematic configuration of the cap shown in FIG. 16.
[FIG. 17B] FIG. 17B is a plan view of the cap shown in FIG. 17A.
[FIG. 17C] FIG. 17C is a front view of the cap shown in FIG. 17A.
[FIG. 17D] FIG. 17D is a cross-sectional view taken along a line A-A' of the cap shown in FIG. 17A.
[FIG. 18A] FIG. 18A is a front view showing an example of a schematic configuration of a medical container having an example of a male connector C (male connector 120) of the present invention to which an example of a female connector A (female connector 125) of the present invention is to be connected.
[FIG. 18B] FIG. 18B is a bottom view of the medical container shown in FIG. 18A.
[FIG. 19A] FIG. 19A is a partially enlarged perspective view of the medical container of FIG. 18A.
[FIG. 19B] FIG. 19B is a partially enlarged front view of the medical container of FIG. 18A.
[FIG. 19C] FIG. 19C is a partially enlarged bottom view of the medical container of FIG. 18A (it should be noted that a container main section is omitted).
[FIG. 20A] FIG. 20A is a perspective view showing a schematic configuration of the female connector 125 to be connected to the male connector 120 shown in FIG. 18A.
[FIG. 20B] FIG. 20B is a plan view of the female connector 125 shown in FIG. 20A.
[FIG. 20C] FIG. 20C is a front view of the female connector 125 shown in FIG. 20A
[FIG. 20D] FIG. 20D is a cross-sectional view taken along a line A-A' of the female connector 125 shown in FIG. 20A.
[FIG. 20E] FIG. 20E is a cross-sectional view taken along a line B-B' of the female connector 125 shown in FIG. 20A.
[FIG. 21] FIG. 21 is a cross-sectional view illustrating a state of the connection between the male connector 120 shown in FIG. 19A and the female connector 125 shown in FIG. 20A.
[FIG. 22A] FIG. 22A is a perspective view illustrating a state of the connection between the male connector 120 shown in FIG. 19A and the female connector 125 shown in FIG. 20A.
[FIG. 22B] FIG. 22B is a perspective view illustrating a state of the connection between the male connector 120 shown in FIG. 19A and the female connector 125 shown in FIG. 20A.
[FIG. 23] FIG. 23 is a cross-sectional view illustrating a state in which the male connector 120 shown in FIG. 19A and the female connector 125 shown in FIG. 20A are connected to each other.
[FIG. 24] FIG. 24 is a cross-sectional view illustrating another example of the female connector of the present invention.
[FIG. 25] FIG. 25 is a front view of another example of the male connector of the present invention to be connected to the female connector shown in FIG. 24.
[FIG. 26A] FIG. 26A is a front view showing an example of a schematic configuration of a medical container having an example of a male connector E (male connector 180) of the present invention to which an example of a female connector D (female connector 85) is to be connected.
[FIG. 26B] FIG. 26B is a bottom view of the medical container shown in FIG. 26A.
[FIG. 27A] FIG. 27A is a partially enlarged perspective view of the medical container of FIG. 26A.
[FIG. 27B] FIG. 27B is a partially enlarged front view of the medical container of FIG. 26A.
[FIG. 27C] FIG. 27C is a partially enlarged bottom view of the medical container of FIG. 26A (it should be noted that a container main section is omitted).
[FIG. 28A] FIG. 28A is a perspective view showing a schematic configuration of the female connector 85 to be connected to the male connector 180 shown in FIG. 26A.
[FIG. 28B] FIG. 28B is a plan view of the female connector 85 shown in FIG. 28A.
[FIG. 28C] FIG. 28C is a front view of the female connector 85A shown in FIG. 28A.
[FIG. 28D] FIG. 28D is a cross-sectional view taken along a line A-A' of the female connector 85 shown in FIG. 28A.
[FIG. 28E] FIG. 28E is a cross-sectional view taken along a line B-B' of the female connector 85 shown in FIG. 28A.
[FIG. 29] FIG. 29 is a cross-sectional view illustrating a state of the connection between the male connector 180 shown in FIG. 27A and the female connector 85 shown in FIG. 28A.
[FIG. 30A] FIG. 30A is a perspective view illustrating a state of the connection between the male connector 180 shown in FIG. 27A and the female connector 85 shown in FIG. 28A.
[FIG. 30B] FIG. 30B is a perspective view illustrating a state of the connection between the male connector 180 shown in FIG. 27A and the female connector 85 shown in FIG. 28A.

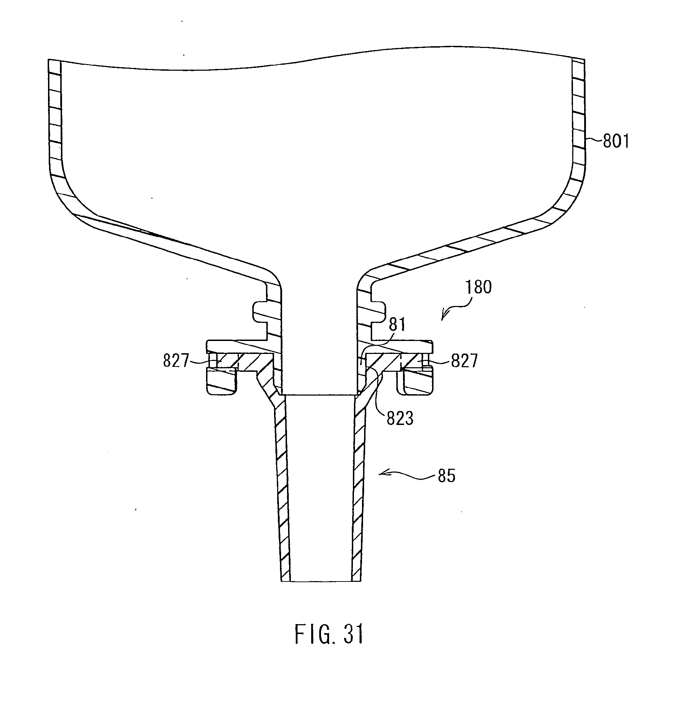

[FIG. 31] FIG. 31 is a cross-sectional view illustrating a state in which the male connector 180 shown in FIG. 27A and the female connector 85 shown in FIG. 28A are connected to each other.
[FIG. 32] FIG. 32 is a cross-sectional view showing schematic configurations of an example of a conventional male connector and an example of a conventional female connector.
[FIG. 33] FIG. 33 is a perspective view of an example of a handle of the conventional female connector.
[FIG. 34] FIG. 34 is a perspective view showing an example of a schematic configuration of the conventional male connector.

### Description of the Invention

In a preferred example of the above-mentioned female connector A of the present invention, when the female connector A is connected to the male connector Q or the male connector C, an outer circumferential surface of the tube-shaped section is in contact with an inner circumferential surface of the tubular section. The female connector A further includes a medium-diameter tube-shaped section, at least a part of an inner circumferential surface of which is in contact with an outer circumferential surface of the tubular section and one end of which is connected to the tube-shaped section through the bottom section. In this case, the medium-diameter tube-shaped section functions so as to press the tubular section against the tube-shaped section side, which further enhances the liquid-tightness of the connection structure of the female connector A and the male connector Q or the male connector C.

A preferred example of the female connector A of the present invention further includes a large-diameter tube-shaped section that is placed around the medium-diameter tube-shaped section so that a space is formed between the medium-diameter tube-shaped section and the large-diameter tube-shaped section, and one end of which is connected to the medium-diameter tube-shaped section through the bottom section. The thickness of the large-diameter tube-shaped section is larger than that of the medium-diameter tube-shaped section, and the brim section is connected to the medium-diameter tube-shaped section through the large-diameter tube-shaped section. In this case, by reducing the thickness of the medium-diameter tube-shaped section to provide the medium-diameter tube-shaped section with slight flexibility the insertion resistance generated when the tubular section is inserted between the medium-diameter tube-shaped section and the tube-shaped section can be reduced, and the strength of the female connector A can be ensured.

A preferred example of the female connector A of the present invention further includes a cross-linking section that is placed between the medium-diameter tube-shaped section and the large-diameter tube-shaped section and partially connects the medium-diameter tube-shaped section to the large-diameter tube-shaped section. In this case, the strength of the medium-diameter tube-shaped section having a relatively smaller thickness can be enhanced. Further, the flexibility of the medium-diameter tube-shaped section having a relatively small thickness can be ensured, and the strength of the medium-diameter tube-shaped section can be held, by adjusting the number of the cross-linking sections.

A preferred example of the female connector A of the present invention further includes a grasping section that protrudes outward from an outer circumferential surface of the large-diameter tube-shaped section. In this case, the rotation of the female connector A can be performed easily.

When the engagement tab of the male connector Q includes an engagement chip and a first lock protrusion formed on a surface of the engagement chip opposite to a surface of the engagement chip on the tube-shaped section side, in a preferred example of the female connector A of the present invention, a surface of the engagement wall facing the engagement tab when the female connector A is connected to the male connector Q includes a first lock concave portion that is connected to the inclined surface and in which the first lock protrusion having slid over the inclined surface is to be stored. In this case, it can be confirmed that the engagement tab and the engagement wall are engaged with each other sufficiently, based on the vibration transmitted to the hand immediately after the first lock protrusion is stored in the first lock concave portion.

In a preferred example of the female connector A of the present invention, the engagement tab of the male connector Q or the male connector C includes a plane that slides over an inclined surface during the connection between the female connector A and the male connector Q or the male connector C and that is parallel to the inclined surface. In this case, the degradation of the engagement tab involved in sliding, such as the scraping off of a portion of the engagement tab, which is in contact with the inclined surface, is suppressed.

In a preferred example of the female connector A of the present invention, when the inclined surface is defined as a first inclined surface, a surface of the engagement wall facing the engagement tab has a stepped structure including the first inclined surface, a second inclined surface, and a first stepped surface extending between the first inclined surface and the second inclined surface, and when the female connector A is connected to the male connector C, the engagement tab of the male connector C includes a third inclined surface opposed to the first inclined surface, a fourth inclined surface opposed to the second inclined surface, and a second stepped surface opposed to the first stepped surface. In this case, since the contact area between the engagement wall and the engagement tab is large, a stress acting in a direction opposite to the rotation direction of the female connector A during the engagement is dispersed, and the damage to the engagement wall and/or engagement tab due to the concentration of a stress, etc. can be suppressed. The male connector C of the present invention to be connected to the female connector A in this embodiment includes: a tubular section; a platform in which the tubular section is provided so as to protrude from a center of one principal surface; and an engagement tab protruding outward from an outer circumferential surface of the platform, wherein the engagement tab includes a third inclined surface opposed to the first inclined surface, a fourth inclined surface opposed to the second inclined surface, and a second stepped surface opposed to the first stepped surface, when the female connector A is connected to the male connector C.

In the case where a surface of the engagement wall facing the engagement tab of the female connector B when the female connector B is connected to the male connector P of the present invention includes a second lock protrusion, in a preferred example of the male connector P of the present invention, the engagement tab includes a second lock concave portion in which the second lock protrusion having slid over the inclined surface is to be stored. In this case, it can be confirmed that the engagement tab and the engagement wall are engaged with each other sufficiently, based on the vibration transmitted to the hand immediately after the second lock protrusion is stored in the second lock concave portion.

When the male connector P is not connected to the female connector B, an opening of the tubular section of the male connector P may be closed with a cap.

The cap includes members having the same structures as those of the tube-shaped section, the bottom section, and the brim section of the female connector B, and a sealing bottom section that closes the opening of the tubular section of the male connector P when the cap is attached to the male connector P, in place of the liquid-sending tube connecting section of the female connector B.

Hereinafter, the present invention will be described in detail by way of preferred embodiments in a transintestine nutrition therapy through a nasotracheal tube. It should be noted that the embodiments merely are embodied examples of the present invention, and the present invention is not limited thereto.

### (Embodiment 1)

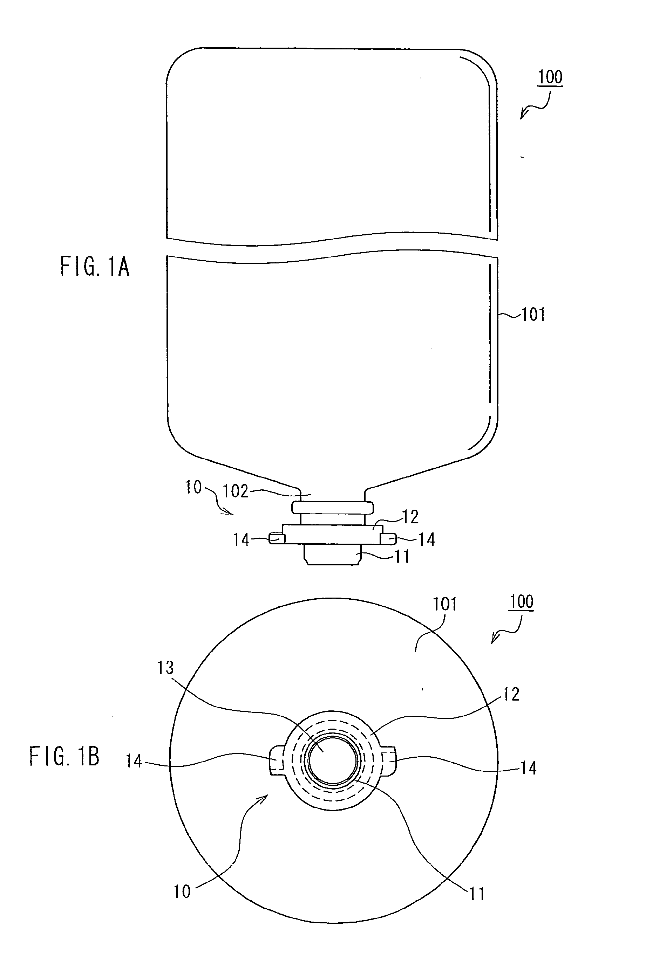

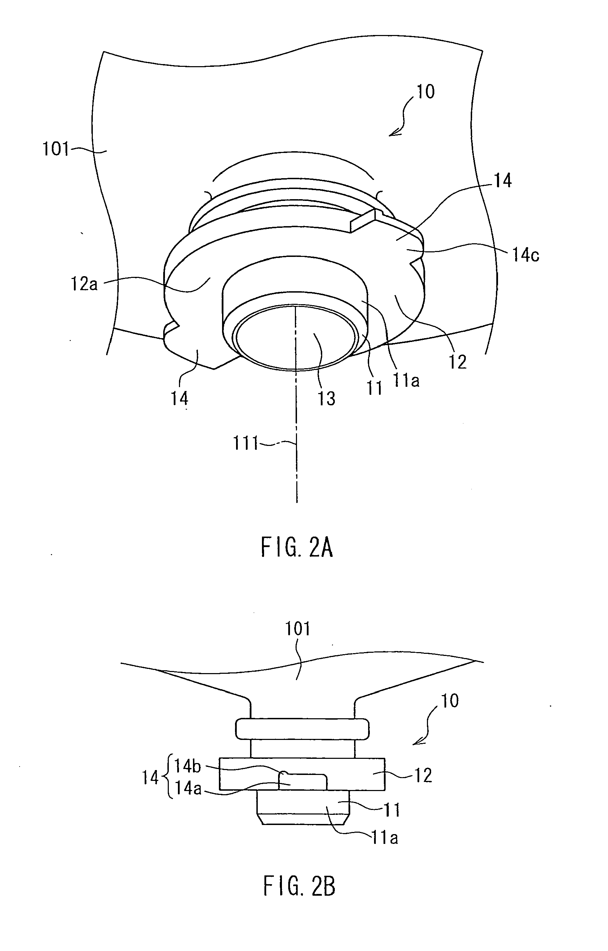

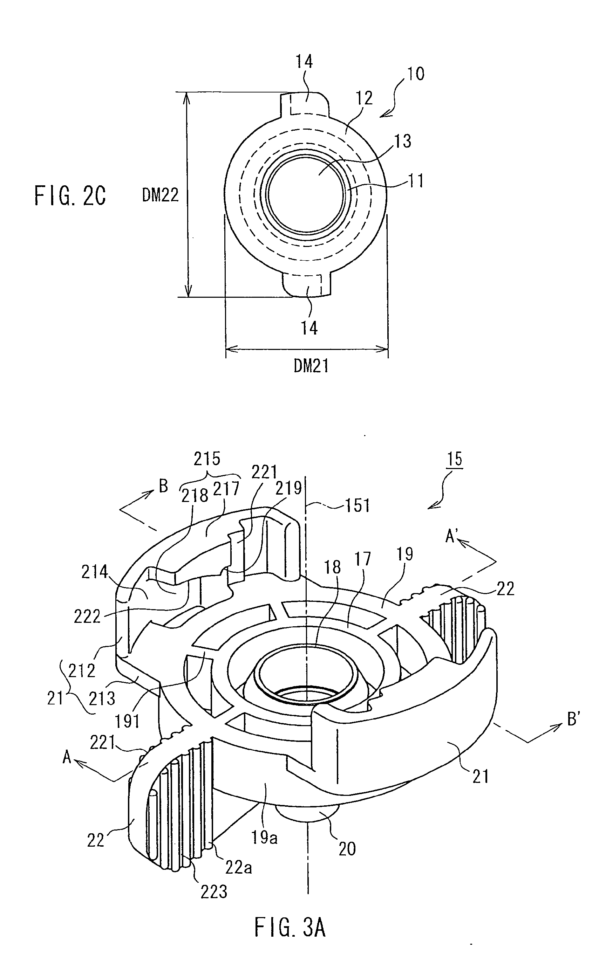

FIG. 1A is a front view showing a schematic configuration of an example of a medical container (medical container 100) including an example of a male connector Q (male connector 10) to which an example of a female connector A (female connector 15) of the present invention is to be connected. FIG. 1B is a bottom view of the medical container shown in FIG. 1A. FIG. 2A is a partially enlarged perspective view of the medical container of FIG. 1A. FIG. 2B is a partially enlarged front view of the medical container of FIG. 1A. FIG. 2C is a partially enlarged bottom view of the medical container of FIG. 1A (it should be noted that a container main section is not shown). In FIG. 2A, alternate long and short dashed lines 111 indicate a center axis of the male connector 10. The direction of the center axis 111 is defined as an up-and-down direction, and an upper side on the drawing surface (a container main section 101 side of the medical container 100) of FIG. 2A is referred to as an "upper side" and a lower side on the drawing surface (side to which the female connector 15 is to be connected) is referred to as a "lower side".

The medical container 100 includes the container main section 101 filled with a liquid-like substance and having a hollow cylindrical port 102 for injecting the liquid-like substance into the container main section 101 or taking out the liquid-like substance therefrom, and the male connector 10. In the example shown in FIG. 1A, the male connector 10 is integrated with the port 102 during molding. The male connector 10 is made of a material having a relatively high hardness, and is formed using, for example, polyolefin resin such as polypropylene or polyethylene, polycarbonater resin, etc. although not limited thereto.

The hardness of the male connector 10 measured based on JIS-K7202 is preferably R40 to R140, and more preferably R50 to R100.

The male connector 10 has a platform 12. The platform 12 is provided with a tubular section 11 protruding downward from the center of one principal surface of the platform 12. In the tubular section 11 and the platform 12, a through-hole 13 through which the liquid-like substance in the container main section 101 flows out is formed. The platform 12 has a pair of engagement tabs 14 protruding outward from an outer circumferential surface of the platform 12. The pair of engagement tabs 14 protrude, for example, at symmetrical positions with respect to the center axis 111 and in a radial direction with respect to the center axis 111.

As shown in FIG. 2B, each engagement tab 14 has an engagement chip 14a extending substantially in a horizontal direction and a first lock protrusion 14b formed at one end of the engagement chip 14a so as to protrude upward. As shown in FIG. 2A, a lower surface 12a of the platform 12 is a flat surface, and the lower surface 12a and lower surfaces 14c of the pair of engagement tabs 14 are present on the same plane. As shown in FIG. 2C, the outer diameter of the platform 12 is DM21, and the distance between tops of the pair of engagement tabs 14 is DM22 (DM22 > DM21).

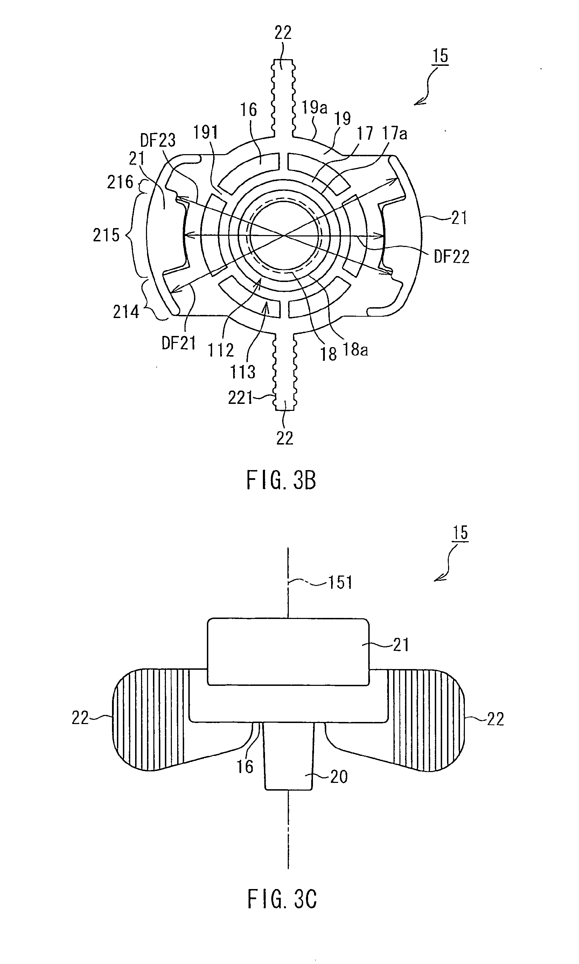

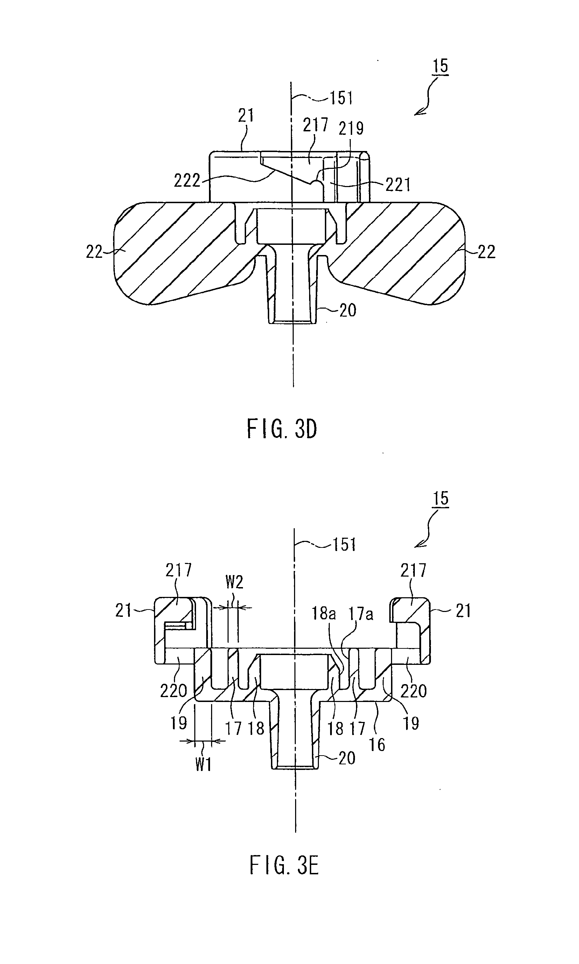

FIG. 3A is a perspective view showing a schematic configuration of an example of the female connector 15 to be connected to the male connector 10. FIG. 3B is a plan view of the female connector 15 shown in FIG. 3A. FIG. 3C is a front view of the female connector 15 shown in FIG. 3A. FIG. 3D is a cross-sectional view taken along an A-A' line of the female connector 15 shown in FIG. 3A. FIG. 3E is a cross-sectional view taken along a B-B' line of the female connector 15 shown in FIG. 3A.

As shown in FIGS. 3A to 3E, the female connector 15 includes a bottom section 16, a tube-shaped section 18, a medium-diameter tube-shaped section 17, a large-diameter tube-shaped section 19, a liquid-sending tube connecting section 20, brim sections 21, and grasping sections 22. Alternate long and short dashed lines 151 indicate a center axis of the tube-shaped section 18, which is matched with the center axis of the female connector 15, the tube-shaped section 18, the medium-diameter tube-shaped section 17, the large-diameter tube-shaped section 19, and the liquid-sending tube connecting section 20. The direction of the center axis 151 is defined as an up-and-down direction, and an upper side on the drawing surface (side to be connected to the male connector 10 described later) of FIG. 3A is referred to as an "upper side" and a lower side on the drawing surface is referred to as a "lower side" (side to which a liquid-sending tube is to be connected).

As shown in FIG. 3E, the tube-shaped section 18 is provided so as to protrude from a surface of the bottom section 16 opposite to a surface thereof on the liquid-sending tube connecting section 20. One end of the tube-shaped section 18 is connected to the liquid-sending tube connecting section 20 through the bottom section 16. The outer diameter of the tube-shaped section 18 is equal to or slightly smaller than the inner diameter of the tubular section 11. Therefore, an outer circumferential surface 18a (see FIG. 3B) of the tube-shaped section 18 comes into tight contact with the inner circumferential surface of the tubular section 11 when the female connector 15 and the male connector 10 are connected to each other. Thus, the male connector 10 and the female connector 15 can be connected to each other reliably without causing the leakage of the liquid-like substance.

The medium-diameter tube-shaped section 17 is placed around the tube-shaped section 18, and one end of the medium-diameter tube-shaped section 17 is connected to the tube-shaped section 18 through the bottom section 16. An annular space 112 (see FIG. 3B) is present between an inner circumferential surface 17a of the medium-diameter tube-shaped section 17 and the outer circumferential surface 18a of the tube-shaped section 18. When the female connector 15 and the male connector 10 are connected to each other, the medium-diameter tube-shaped section 17 functions in such a manner that the inner circumferential surface 17a (see FIG. 3B, etc.) comes into contact with an outer circumferential surface of the tubular section 11 to press the tubular section 11 against the tube-shaped section 18 side. In the female connector 15, the medium-diameter tube-shaped section 17 is not necessarily required. However, the presence of the medium-diameter tube-shaped section 17 can suppress the leakage of the liquid-like substance more reliably. From the viewpoint of enhancing the contact between the inner circumferential surface 17a of the medium-diameter tube-shaped section 17 and the outer circumferential surface 11a (see FIG. 2A, etc.) of the tubular section 11, the inner diameter of the medium-diameter tube-shaped section 17 may decrease gradually from the lower side (bottom section 16 side) to the upper side (tip end side of the medium-diameter tube-shaped section 17).

The large-diameter tube-shaped section 19 is placed around the medium-diameter tube-shaped section 17 and spaced therefrom. One end of the large-diameter tube-shaped section19 is connected to the tube-shaped section 18 and the medium-diameter tube-shaped section 17 through the bottom section 16. The large-diameter tube-shaped section 19 is placed between the medium-diameter tube-shaped section 17 and the brim sections 21 to connect them to each other. A thickness W1 of the large-diameter tube-shaped section 19 is larger than a thickness W2 of the medium-diameter tube-shaped section 17 (see FIG. 3E). In the female connector 15, the brim sections 21 may be connected directly to the medium-diameter tube-shaped section 17, and the large-diameter tube-shaped section 19 is not necessarily required. However, the presence of the large-diameter tube-shaped section 19 can decrease the insertion resistance generated when the tube-shaped section 18 is inserted in the tubular section 11 and also ensure the strength of the female connector 15, while enhancing liquid-tightness for the reasons described later.

As described above, when the inner diameter of the medium-diameter tube-shaped section 17 decreases gradually from the lower side to the upper side, the contact between the inner circumferential surface 17a of the medium-diameter tube-shaped section 17 and the outer circumferential surface 11a of the tubular section 11 is enhanced to increase liquid-tightness. However, along with the enhancement of contact, the insertion resistance generated when the tubular section 11 is inserted in the medium-diameter tube-shaped section 17 increases. Further, the above-mentioned insertion resistance generated during the rotation of the female connector 15 and/or the male connector 10, which is conducted in order to engage the engagement tabs 14 of the male connector 10 with the brim sections 21 of the female connector 15, also increases. The above-mentioned insertion resistance can be decreased, for example, by reducing the thickness W2 of the medium-diameter tube-shaped section 17 to provide the medium-diameter tube-based section 17 with some flexibility. However, on the other hand, the strength of the medium-diameter tube-shaped section 17 decreases. When the brim sections 21 are connected directly to the thin medium-diameter tube-shaped section 17, the durability of the female connector 15 with respect to the above-mentioned rotation is degraded.

If the female connector 15 has the large-diameter tube-shaped section 19, which is connected to the medium-diameter tube-shaped section 17 and the tube-shaped section 18 through the bottom section 16 and is larger in thickness than the tube-shaped section 18, even when the thickness W2 of the medium-diameter tube-shaped section 17 is reduced so as to decrease the above-mentioned insertion resistance, the strength of the female connector 15 can be ensured. Further, when the female connector 15 has a certain size, the female connector 15 can be grasped easily, which enables the easy rotation. From such a viewpoint, it is preferred that the female connector 15 has the large-diameter tube-shaped section 19.

It is preferred that the female connector 15 has a plurality of cross-linking sections 191 (see FIG. 3B) that are provided between the medium-diameter tube-shaped section 17 and the large-diameter tube-shaped section 19 and that extend in a radial direction with respect to the center axis 151 to connect partially the outer circumferential surface of the medium-diameter tube-shaped section 17 and the inner circumferential surface of the large-diameter tube-shaped section 19 to each other. When the female connector 15 has the plurality of cross-linking sections 191, the strength of the medium-diameter tube-shaped section 17 is enhanced further. The number of the cross-linking sections 191 is selected appropriately depending upon a material and the like of the female connector 15 so that the flexibility of the medium-diameter tube-shaped section 17 is ensured and the strength of the medium-diameter tube-shaped section 17 is held. It is more preferred that the plurality of cross-linking sections 191 are placed at an equal interval in a circumferential direction, that is, a space 113 (see FIG. 3B) between the medium-diameter tube-shaped section 17 and the large-diameter tube-shaped section 19 is divided at an equal interval in the circumferential direction from the viewpoint of ensuring the flexibility of the medium-diameter tube-shaped section 17 and holding the strength of the medium-diameter tube-shaped section 17.

A pair of the grasping sections 22 protruding outward from the outer circumferential surface 19a of the large-diameter tube-shaped section 19 (in a direction orthogonal to the center axis 151) are formed around the large-diameter tube-shaped section 19. Regarding the rotation conducted so as to engage the engagement tabs 14 with the brim sections 21, the female connector 15 is rotated with the pair of grasping sections 22 grasped. Although the pair of grasping sections 22 are not necessarily required, the presence of the pair of grasping sections 22 in the female connector 15 facilitates the above-mentioned rotation.

It is preferred that each grasping section 22 is placed between the brim sections 21 adjacent to each other in the circumferential direction for the reason that the grasping section 22 can be grasped easily and the rotation can be conducted easily, and it is more preferred that each grasping section 22 is placed so that the distance from each of the brim sections 21 adjacent to each other in the circumferential direction becomes equal. Although there is no particular limit to the shape of each grasping section 22, it is preferred that a pair of principal surfaces 221, 223 of each grasping section 22 are parallel to an up-and-down direction (the direction of the center axis 151 is defined as the up-and-down direction), as shown in FIG. 3A, for the reason that the grasping sections 22 can be grasped easily and the rotation can be conducted easily. Further, it is more preferred that a plurality of ribs 22a for preventing slipping are formed on at least one of the pair of principal surfaces 221, 223, and it is still more preferred that the longitudinal direction of the ribs 22a is parallel to the up-and-down direction.

The pair of brim sections 21 are formed around and on the upper side of the large-diameter tube-shaped section 19. The pair of brim sections 21 are symmetrical with respect to the center axis 151. The brim section 21 includes an arc-shaped wall 212 substantially along the cylindrical surface with the center axis 151 being a center axis, and a bridge section 213 that extends in a direction perpendicular to the center axis 151 and connects the lower end of the arc-shaped wall 212 to the upper end of the large-diameter tube-shaped section 19.

The arc-shaped wall 212 includes a passage region 214, an engagement region 215, and a non-passage region 216 (see FIG. 3B) in accordance with the difference in shape of the inner circumferential surface opposed to the center axis 151. The inner circumferential surface of the passage region 214 is a part of a cylindrical surface with a diameter DF21, and the inner circumferential surface of the non-passage region 216 is a part of a cylindrical surface with a diameter DF23 (DF23 < DF22). The engagement region 215 between the passage region 214 and the non-passage region 216 includes an engagement wall 217 at an upper end, which extends in the circumferential direction so as to connect the upper end of the passage region 214 to the upper end of the non-passage region 216. The inner circumferential surface of the engagement wall 217 opposed to the center axis 151 is a part of a cylindrical surface with a diameter DF22 (DF22 < DF21). Due to the presence of the engagement wall 217 in the engagement region 215, a region between the engagement wall 217 and the bridge section 213 is dented in a concave shape. More specifically, the region between the engagement wall 217 and the bridge section 213 is recessed relatively from the engagement wall 217 with respect to the center axis to form a concave portion 218. The non-passage region 216 adjacent to the engagement region 215 in the circumferential direction includes a convex portion 221that protrudes relative to the concave portion 218. On the lower surface of the engagement wall 217 on the non-passage region 216 side (an end portion on the non-passage region 216 side of a surface of the engagement wall 217 on the concave portion 218 side, an end portion on the non-passage region 216 side of a surface of the engagement wall 217 facing the bridge section 213), a lock concave portion 219 dented in a concave shape is formed. In a portion of the bridge section 213, facing the engagement wall 217, athrough-hole 220 (see FIG. 3E) passing through the bridge section 213 in a thickness direction thereof is formed however, the through-hole 220 need not be provided.

A portion of a surface of the engagement wall 217 on the concave portion 218 side (surface facing the bridge section 213), away from the convex portion 221 relative to a portion in which the first lock concave portion 219 is formed, specifically, a region from one end of the surface of the engagement wall 215 on the concave portion 218 side, away from the convex portion 221, to the first lock concave portion 219 includes an inclined surface 222. The inclined surface 222 is inclined gradually so that the distance from the bridge section 213 (in the case where the through-hole 220 is formed in the bridge section 213, an imaginary surface in the same plane as that of the bridge section 213, facing the inclined surface 222) decreases from one end away from the convex portion 221 to the first lock concave portion 219.

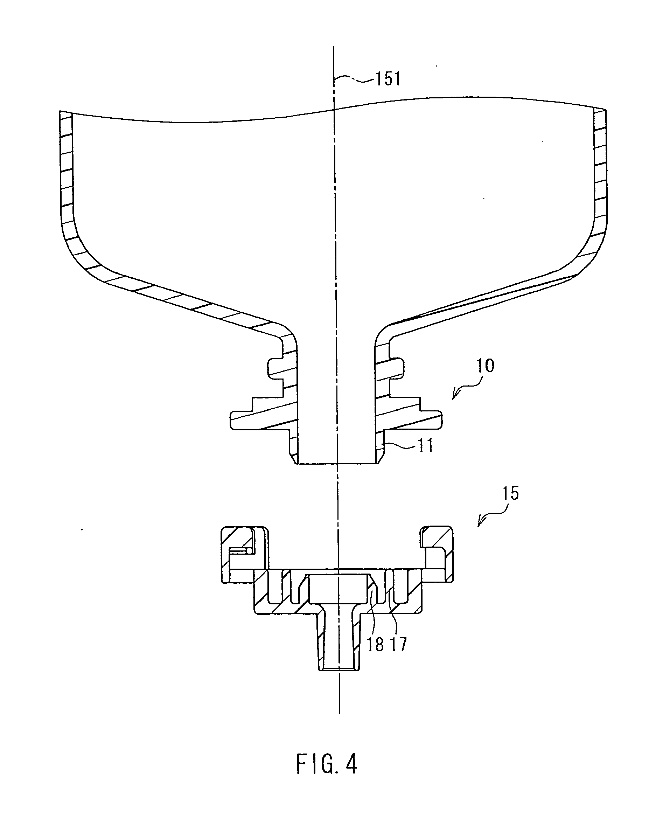

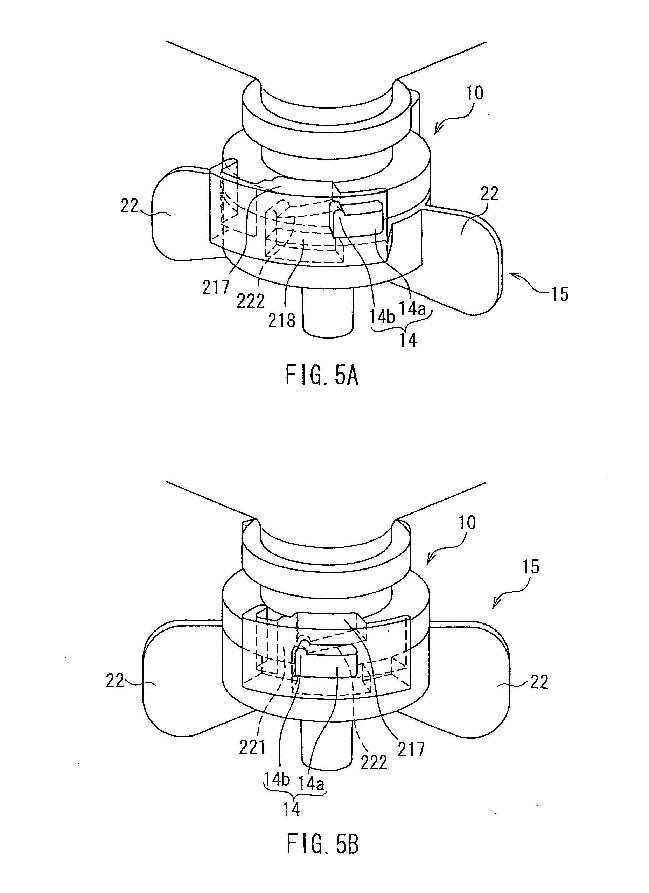

Next, a method for connecting the female connector 15 to the male connector 10 will be described. As shown in FIG. 4, the tubular section 11 of the male connector 10 is inserted in the space between the tube-shaped section 18 and the medium-diameter tube-shaped section 17 of the female connector 15. Then, for example, when the female connector 15 is rotated slightly around the center axis, the first lock protrusion 14b of the engagement tab 14 comes into contact with the inclined surface 222 of the engagement wall 217, as shown in FIG. 5A.

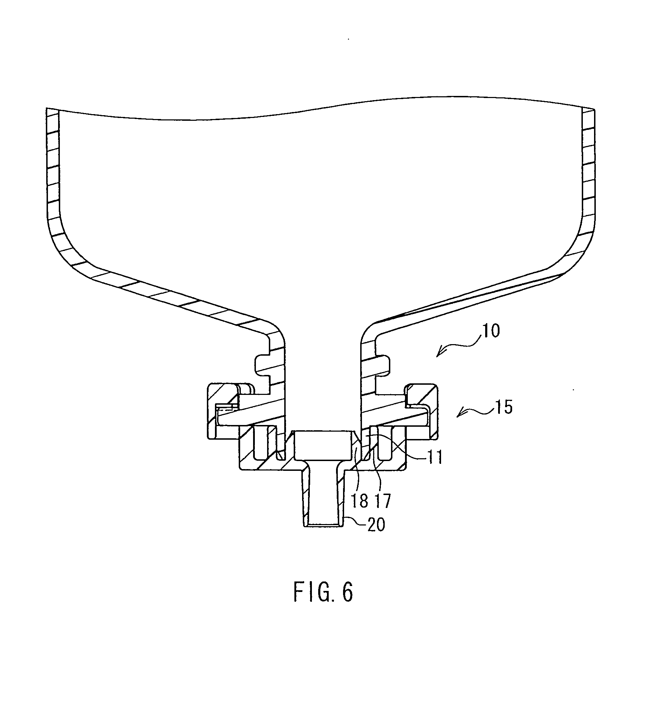

Then, the female connector 15 is rotated around the center axis with respect to the male connector 10 with the grasping sections 22 grasped. As shown in FIGS. 5A and 5B, the first lock protrusion 14b slides over the inclined surface 222 while being pressed against the inclined surface 222 along with the rotation of the female connector 15, and the engagement tab 14 mounts the inclined surface 222 in the sliding direction of the engagement tab 14 with respect to the engagement wall 217. That is, the surface of the engagement wall 217 facing the engagement tab 14 includes the inclined surface 222 which the engagement tab 14 mounts in the sliding direction of the engagement tab 217 with respect to the engagement wall 217 during the connection between the female connector 15 and the male connector 10. Therefore, a force acting so that the tubular section 11 of the male connector 10 is pushed in between the tube-shaped section 18 and the medium-diameter tube-shaped section 17 of the female connector 15 (force at which the male connector 10 and the female connector 15 approach each other) is provided to the male connector 10 and the female connector 15, along with the rotation of the female connector 15. Therefore, the engagement tab 14 is stored in the concave portion 218, and the tubular section 11 is inserted more deeply between the tube-shaped section 18 and the medium-diameter tube-shaped section 17, whereby a state shown in FIG. 6 is obtained.

Thus, even when both the male connector 10 and the female connector 15 are made of a material having a hardness higher than that of a material having flexibility and elasticity such as natural rubber, or synthetic rubber, the tube-shaped section 18 can be inserted easily in a deeper portion of the tubular section 11, and high liquid-tightness obtained by the tight contact between the inner circumferential surface of the tubular section 11 and the outer circumferential surface of the tube-shaped section 18 also can be ensured. Further, since the tube-shaped section 18 can be inserted easily in a deeper portion of the tubular section 11, the sliding resistance of the engagement tab 14 with respect to the inclined surface 222 also can be decreased. Further, since the female connector 15 is made of a material having a hardness higher than that of a material having flexibility and elasticity such as natural rubber, or synthetic rubber, a liquid-sending tube having flexibility can be connected easily to the liquid-sending tube connecting section 20 of the female connector 15, and in particular, the liquid-sending tube can be connected easily to the liquid-sending tube connecting section by a machine. Accordingly, a cost also can be reduced.

The female connector 15 is provided with the convex portion 221 (see FIG. 3A) adjacent to the concave portion 218 in which the engagement tab 14 is to be stored. Therefore, if the engagement tab 14 of the male connector 10 is allowed to enter the concave portion 218 and move further until coming into contact with the convex portion 221, the engagement tab 14 and the engagement wall 217 can be engaged with each other reliably.

It is preferred that the rotation of the female connector 15 with respect to the male connector 10 is regulated by the contact of the engagement tab 14 of the male connector 10 with the convex portion 221 of the female connector 15. Thus, the engagement tab 14 and the engagement wall 217 can be engaged with each other reliably by a simple operation of rotating the female connector 15 with respect to the male connector 10 until the rotation is regulated.

The hardness of the male connector 10 measured based on JIS-K7202 is preferably R40 to R140, more preferably R50 to R100, for the reason that the engagement tab 14 is unlikely to be scraped off during the engagement of the engagement tab 14 with the engagement wall 217.

The hardness of the female connector 15 measured based on JIS-K7202 is preferably R40 to R140, more preferably R50 to R100, from the viewpoint of enhancing the connection operability of the liquid-sending tube having flexibility with respect to the liquid-sending tube connecting section:

### (Embodiment 2)

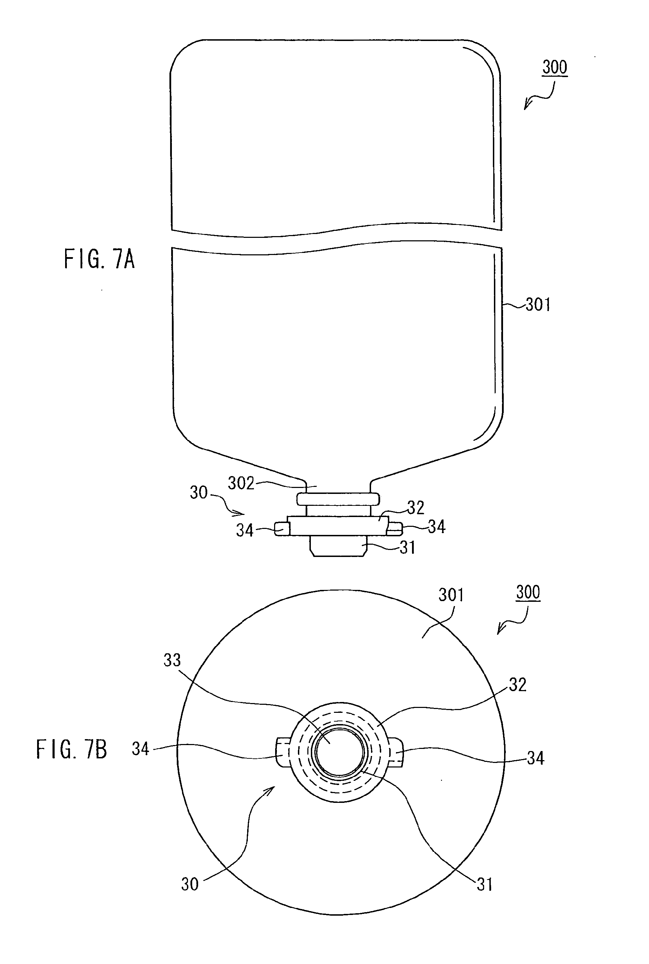

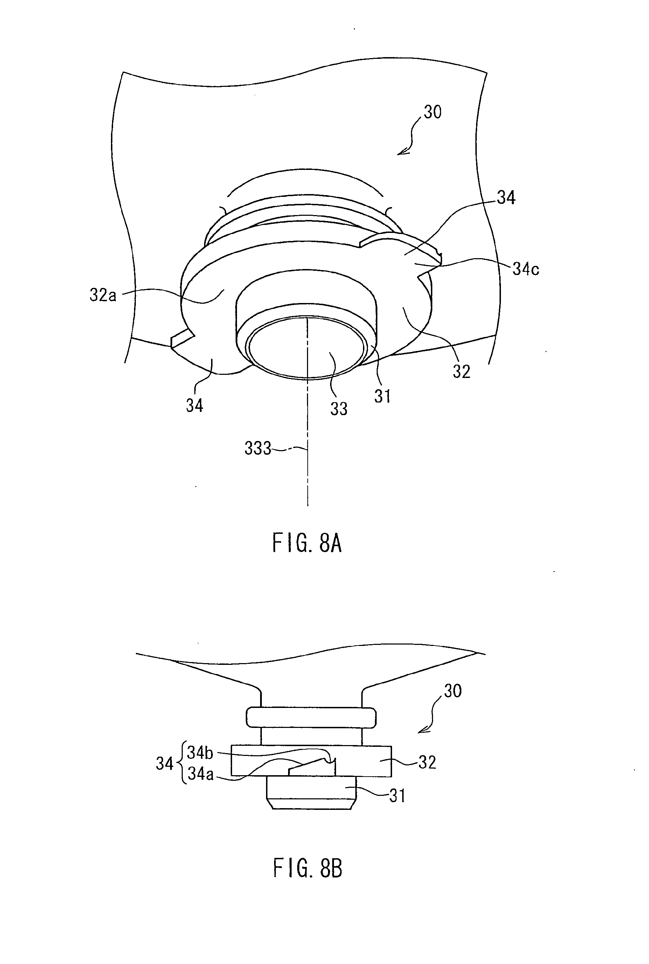

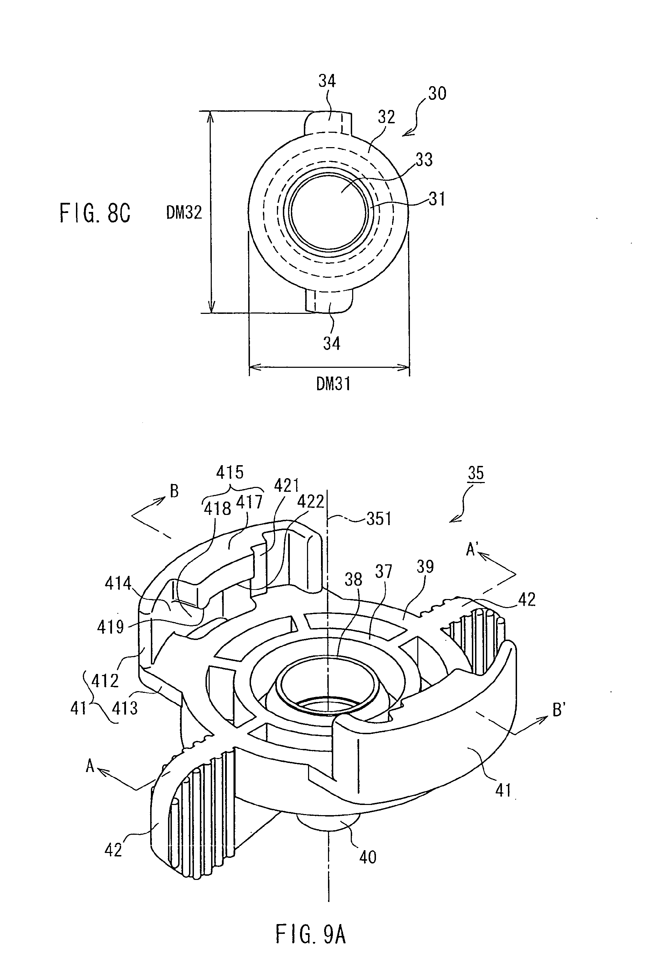

FIG. 7A is a front view showing a schematic configuration of an example of a medical container (medical container 300) including an example of a male connector P (male connector 30) of the present invention to which an example of a female connector B (female connector 35) is to be connected. FIG. 7B is a bottom view of the medical container shown in FIG. 7A. FIG. 8A is a partially enlarged perspective view of the medical container of FIG. 7A. FIG. 8B is a partially enlarged front view of the medical container of FIG. 8A. FIG. 8C is a partially enlarged bottom view of the medical container of FIG. 7A (it should be noted that a container main section is not shown). In FIG. 8A, alternate long and short dashed lines 333 indicate a center axis of the male connector 30. The direction of the center axis 333 is defined as an up-and-down direction, and an upper side on the drawing surface (a container main section 301 side of the medical container 300) of FIG. 8A is referred to as an "upper side" and a lower side on the drawing surface (side to which the female connector 35 is to be connected) is referred to as a "lower side".

The medical container 300 includes the container main section 301 filled with a liquid-like substance and having a hollow cylindrical port 302 for injecting the liquid-like substance into the container main section 301 or taking out the liquid-like substance therefrom, and the male connector 30. In the example shown in FIG. 7A, the male connector 30 is integrated with the port 302 during molding. The material and hardness of the male connector 30 may be the same as those of the medical container 100 described in (Embodiment 1).

The male connector 30 has a platform 32. The platform 32 is provided with a tubular section 31 protruding downward from the center of one principal surface of the platform 32. In the tubular section 31 and the platform 32, a through-hole 33 through which the liquid-like substance in the container main section 301 flows out is formed. The platform 32 has a pair of engagement tabs 34 protruding outward from an outer circumferential surface of the platform 32. The pair of engagement tabs 34 protrude at symmetrical positions with respect to the center axis 333 and in a radial direction with respect to the center axis 333.

As shown in FIG. 8B, an upper surface of each engagement tab 34 (surface opposite to a surface on the tubular section 31 side) includes an inclined surface 34a and a portion dented in a concave shape (second lock concave portion 34b). The height of the inclined surface 34a increases from one end (end on the left side on the drawing surface of FIG. 8B) away from the second lock concave portion 34b of both ends in the circumferential direction of the platform 32 on the upper surface of the engagement tab 34 to the second lock concave portion 34b. That is, the inclined surface 34a is an inclined surface in which the other end is higher than the above-mentioned one end. As shown in FIG. 8B, the thickness of each engagement tab 34 increases gradually from an end on the left side to the second lock concave portion 34b. As shown in FIG. 8A, a lower surface 32a (see FIG. 8A) of the platform 32 is a flat surface. The lower surface 32a and lower surfaces 34c of a pair of engagement tabs 34 are present on the same plane. The outer diameter of the platform 32 is DM31, and the distance between tops of the pair of engagement tabs 34 is DM32 (DM32 > DM31).

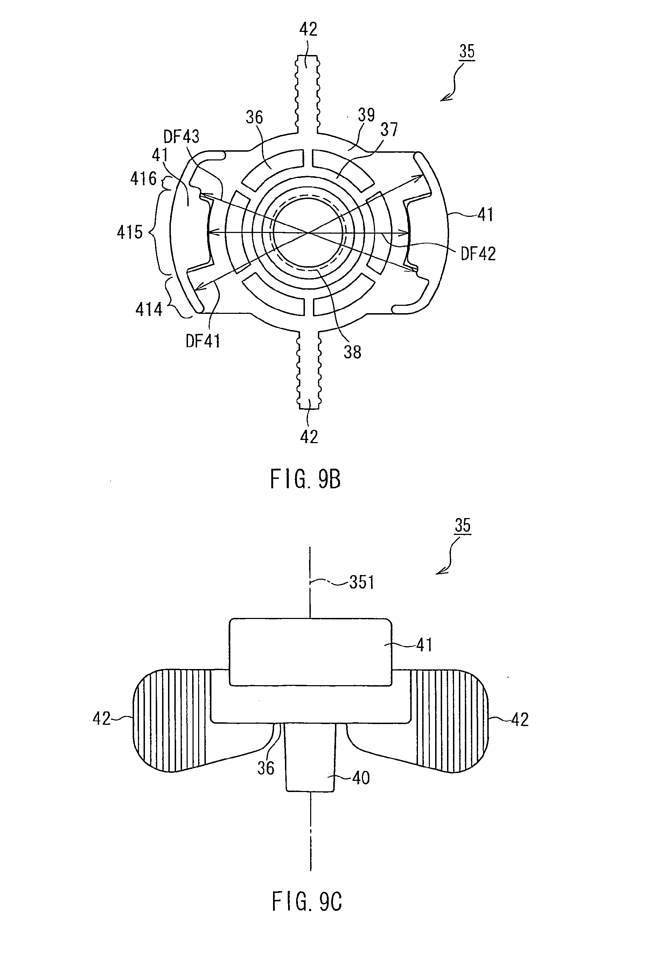

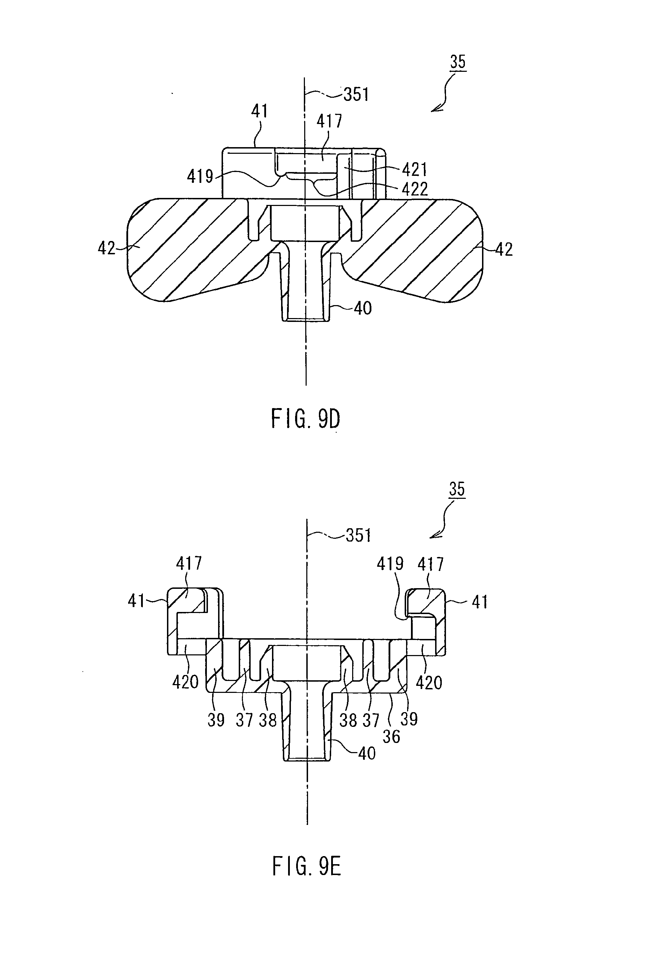

FIG. 9A is a perspective view showing a schematic configuration of an example of the female connector 35 to be connected to an example of the male connector 30 of the present invention. FIG. 9B is a plan view of the female connector 35 shown in FIG. 9A. FIG. 9C is a front view of the female connector 35 shown in FIG. 9A. FIG. 9D is a cross-sectional view taken along an A-A' line of the female connector 35 shown in FIG. 9A. FIG. 9E is a cross-sectional view taken along a B-B' line of the female connector 35 shown in FIG. 9A.

As shown in FIGS. 9A to 9E, the female connector 35 includes a bottom section 36, a tube-shaped section 38, a medium-diameter tube-shaped section 37, a large-diameter tube-shaped section 39, a liquid-sending tube connecting section 40, brim sections 41, and grasping sections 42. Alternate long and short dashed lines 351 indicate a center axis of the tube-shaped section 38, which is matched with the center axis of the female connector 35, the medium-diameter tube-shaped section 37, the large-diameter tube-shaped section 39, and the liquid-sending tube connecting section 40. The direction of the center axis 351 is defined as an up-and-down direction, and an upper side on the drawing surface (side to be connected to the male connector 30) of FIG. 9A is referred to as an "upper side" and a lower side on the drawing surface is referred to as a "lower side" (side to which a liquid-sending tube is to be connected).

The female connector 35 has the same configuration as that of the female connector 15 described in (Embodiment 1), except that the shape of an arc-shaped wall 412 of the brim section 41 is different.

The arc-shaped wall 412 includes a passage region 414, an engagement region 415, and a non-passage region 416 (see FIG. 9B) in accordance with the difference in shape of the inner circumferential surface opposed to the center axis 351. The inner circumferential surface of the passage region 414 is a part of a cylindrical surface with a diameter DF41, and the inner circumferential surface of the non-passage region 416 is a part of a cylindrical surface with a diameter DF43 (DF43 < DF42). The engagement region 415 between the passage region 414 and the non-passage region 416 includes an engagement wall 417 at an upper end, which extends in the circumferential direction so as to connect the upper end of the passage region 414 to the upper end of the non-passage region 416. The inner circumferential surface of the engagement wall 417 opposed to the center axis 351 is a part of a cylindrical surface with a diameter DF42 (DF42 < DF41). Due to the presence of the engagement wall 417 in the engagement region 415, a region between the engagement wall 417 and the bridge section 413 is dented in a concave shape. More specifically, the region between the engagement wall 417 and the bridge section 413 is recessed relatively from the engagement wall 417 with respect to the center axis 351 to form a concave portion 418. The non-passage region 416 adjacent to the engagement region 415 in the circumferential direction includes a convex portion 421 that protrudes relative to the concave portion 418. On the lower surface of the engagement wall 417 on the passage region 414 side (an end portion on the passage region 414 side of a surface of the engagement wall 417 on the concave portion 418 side, an end portion on the passage region 414 side of a surface of the engagement wall 417 facing the bridge section 413), a second lock protrusion 419 protruding downward is formed. In a portion of the bridge section 413, facing the engagement wall 417, a through-hole 420 passing through the bridge section 413 in a thickness direction thereof is formed; however, the through-hole 420 need not be provided.

A portion of a surface of the engagement wall 417 on the concave portion 418 side (surface facing the bridge section 413), closer to the convex portion 421 relative to a portion in which the second lock protrusion 419 is formed, specifically, a region 422 from one end (end of the engagement wall 417 on the non-passage region 416 side) close to the convex portion 421 to the second lock protrusion 419 is substantially horizontal or horizontal.

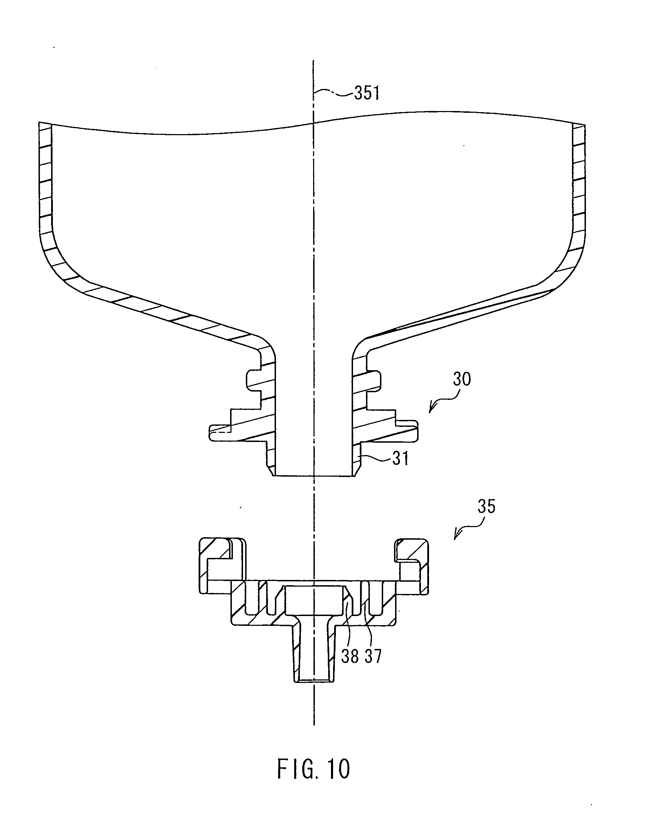

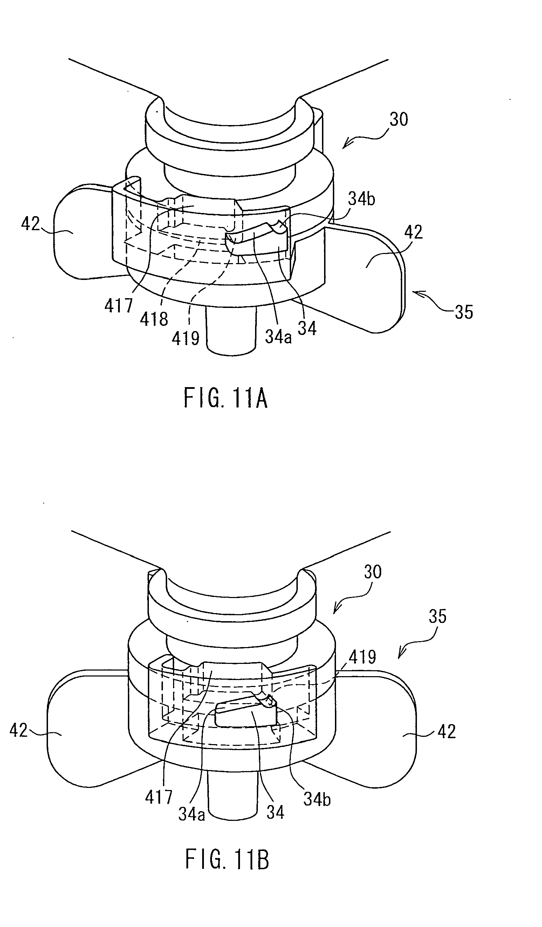

Next, a method for connecting the female connector 35 to the male connector 30 will be described. As shown in FIG. 10, the tubular section 31 of the male connector 30 is inserted in the space between the tube-shaped section 38 and the medium-diameter tube-shaped section 37 of the female connector 35. Then, for example, when the female connector 35 is rotated slightly around the center axis 351, the second lock protrusion 419 of the engagement wall 417 comes into contact with the inclined surface 34a of the engagement tab 34, as shown in FIG. 11A.

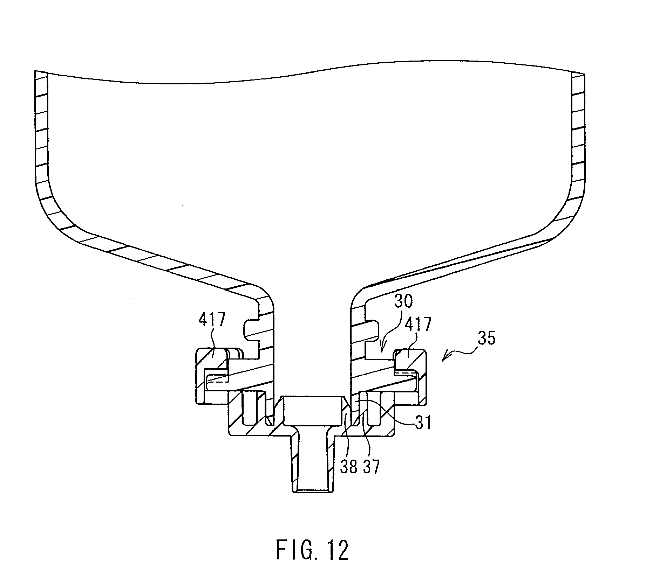

Then, for example, the female connector 35 is rotated around the center axis with respect to the male connector 30 with the grasping sections 42 grasped. The second lock protrusion 419 slides over the inclined surface 34a while being pressed against the inclined surface 34a along with the rotation of the female connector 35 and mounts the inclined surface 34a in the sliding direction of the engagement wall 417 with respect to the engagement tab 34. That is, the surface of the engagement tab 34 facing the engagement wall 417 includes the inclined surface 34a which the engagement wall 417 mounts in the sliding direction of the engagement wall 417 with respect to the engagement tab 34 during the connection between the female connector 35 and the male connector 30. Therefore, a force acting so that the tubular section 31 of the male connector 30 is pushed in between the tube-shaped section 38 and the medium-diameter tube-shaped section 37 of the female connector 35 (force at which the male connector 30 and the female connector 35 approach each other) is provided to the male connector 30 and the female connector 35. Therefore, the engagement tab 34 is stored in the concave portion 418, and the tubular section 31 is inserted more deeply between the tube-shaped section 38 and the medium-diameter tube-shaped section 37, and the second lock protrusion 419 of the engagement wall 417 is stored in the second lock concave portion 34b of the engagement tab 34, whereby the male connector 30 and the female connector 35 assume a state shown in FIG. 12.

Thus, even when both the male connector 30 and the female connector 35 are made of a material having a hardness higher than that of a material having flexibility and elasticity such as natural rubber, or synthetic rubber, the tube-shaped section 38 can be inserted easily in a deeper portion of the tubular section 31, and high liquid-tightness obtained by the tight contact between the inner circumferential surface of the tubular section 31 and the outer circumferential surface of the tube-shaped section 38 also can be ensured. Further, since the tube-shaped section 38 can be inserted easily in a deeper portion of the tubular section 31, the sliding resistance of the second lock protrusion 419 with respect to the inclined surface 34a of the engagement tab 34 also can be decreased. Further, since the female connector 35 is made of a material having a hardness higher than that of a material having flexibility and elasticity such as natural rubber, or synthetic rubber, a liquid-sending tube having flexibility can be connected easily to the liquid-sending tube connecting section 40 of the female connector 35, and the cost also can be reduced. The material and hardness of the female connector 35 and the male connector 30 may be the same as those of the female connector 15 and the male connector 10 described in (Embodiment 1).

The female connector 35 is provided with the convex portion 421 adjacent to the concave portion 418 in which the engagement tab 34 is to be stored. Therefore, if the engagement tab 34 of the male connector 30 is allowed to enter the concave portion 418 and move further until coming into contact with the convex portion 421, the engagement tab 34 and the engagement wall 417 can be engaged with each other reliably.

It is preferred that the rotation of the female connector 35 with respect to the male connector 30 is regulated by the contact of the engagement tab 34 of the male connector 30 with the convex portion 421 of the female connector 35. Thus, the engagement tab 34 and the engagement wall 417 can be engaged with each other reliably by a simple operation of rotating the female connector 35 with respect to the male connector 30 until the rotation is regulated.

### (Embodiment 3)

The male connector 10 to be connected to the female connector 15 described in (Embodiment 1) and an example of the male connector P (male connector 30) described in (Embodiment 2) respectively are integrated with a port of a medical container in the course of molding. However, these male connectors may be connected to the port removably through connection means.

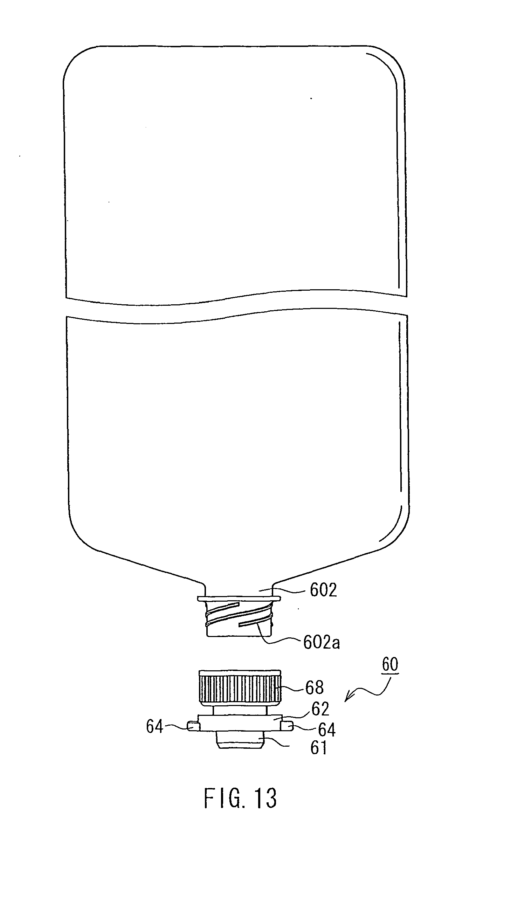

As shown in FIG. 13, a male connector 60 that is an example of the male connector P of the present invention includes a platform 62, a tubular section 61 protruding downward from the center of one principal surface of the platform 62, a cap section 68 placed on a side of the platform 62 opposite to the tubular section 61 side, and a pair of engagement tabs 64 protruding outward from the outer circumferential surface of the platform 62. The platform 62, the tubular section 61, and the engagement tabs 64 may have the same configurations as those of the male connector 30 described in (Embodiment 2). The inner circumferential surface of the cap section 68 is provided with a female thread (not shown) to be screwed to a male thread section 602a of a port 602. The material and hardness of the male connector 60 may be the same as those of the male connector 30 described in (Embodiment 2).

### (Embodiment 4)

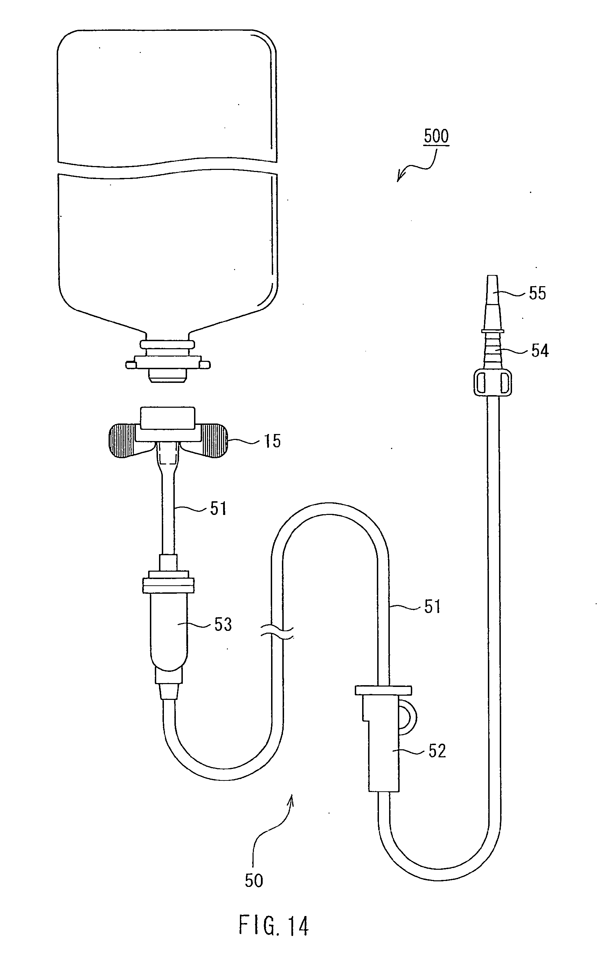

As shown in FIG. 14, an example of the female connector A of the present invention (female connector 15) described in (Embodiment 1) or the female connector 35 described in (Embodiment 2) is used, for example, as a component of a liquid-sending circuit 50 constituting a transintestine nutrient supply set such as a transintestine nutrient supply set 500.

The liquid-sending circuit 50 includes, for example, a flexible liquid-sending tube 51 connected to the female connector 15, a flow rate adjuster 52 for adjusting the flow rate of a liquid-like substance flowing through the liquid-sending tube 51 by pressing a liquid-sending tube 51, a drip tube 53, a connector 54, and a connector cover 55. However, the liquid-sending circuit of the present invention is not limited to the configuration shown in FIG. 14, and further may include components of conventionally known transintestine nutrient supply sets.

The liquid-sending circuit including the female connector 35 described in (Embodiment 2) also may include components of the conventionally known transintestine nutrient supply sets.

### (Embodiment 5)

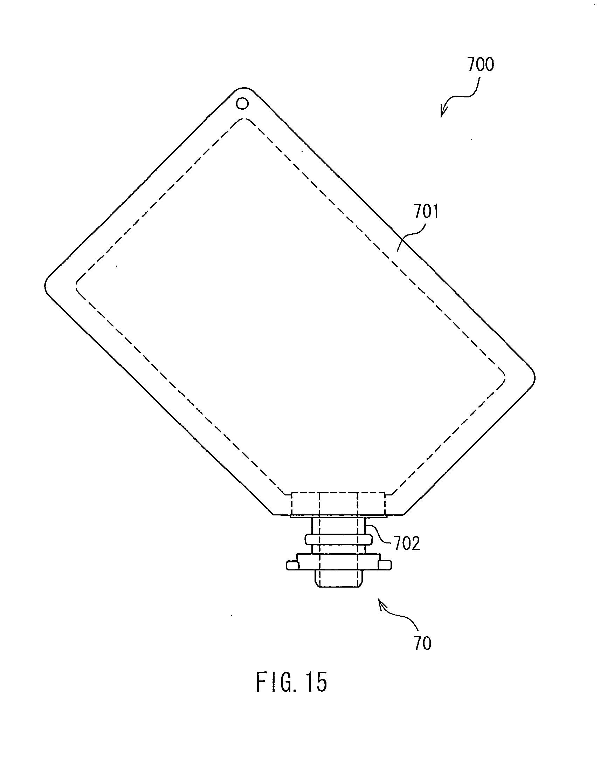

As shown in FIG. 15, in an example of the medical container of the present invention, a container main section 701 may be made of a soft plastic sheet. An example of the male connector P of the present invention (male connector 70) constituting the medical container 700 may have the same configuration as that of the male connector 30 of the present invention described in (Embodiment 2). In this case, the container main section 701 is, for example, a bag-shaped member obtained by overlapping two soft plastic sheets each other and connecting outer peripheral edges thereof to each other by heat sealing (thermobonding). The port 702 and the male connector 70 are made of resin material that is relatively hard, compared with the above-mentioned soft plastic sheet constituting the container main section 701. The material and hardness of the port 702 and the male connector 70 may be the same as those of the male connector 30 described in (Embodiment 2).

There is no particular limit to the material for the soft plastic sheet, and conventionally known soft plastic sheets used in a medical container can be used. Specifically, examples of the material for the soft plastic sheet include a single layer sheet made of vinyl chloride resin, polyethylene, an ethylene - vinyl acetate copolymer, polyester, polybutadiene, polypropylene, polyamide, an ethylene-methacrylate copolymer, or the like, and a multi-layered sheet obtained by layering the single layer sheets. Examples of the specific layer configuration of the multi-layered sheet include nylon/polyethylene, nylon/polypropylene, polyethylene terephthalate/polyethylene, polyethylene terephthalate/polypropylene, polypropylene/polyethylene, and nylon/polypropylene/polyethylene.

### (Embodiment 6)

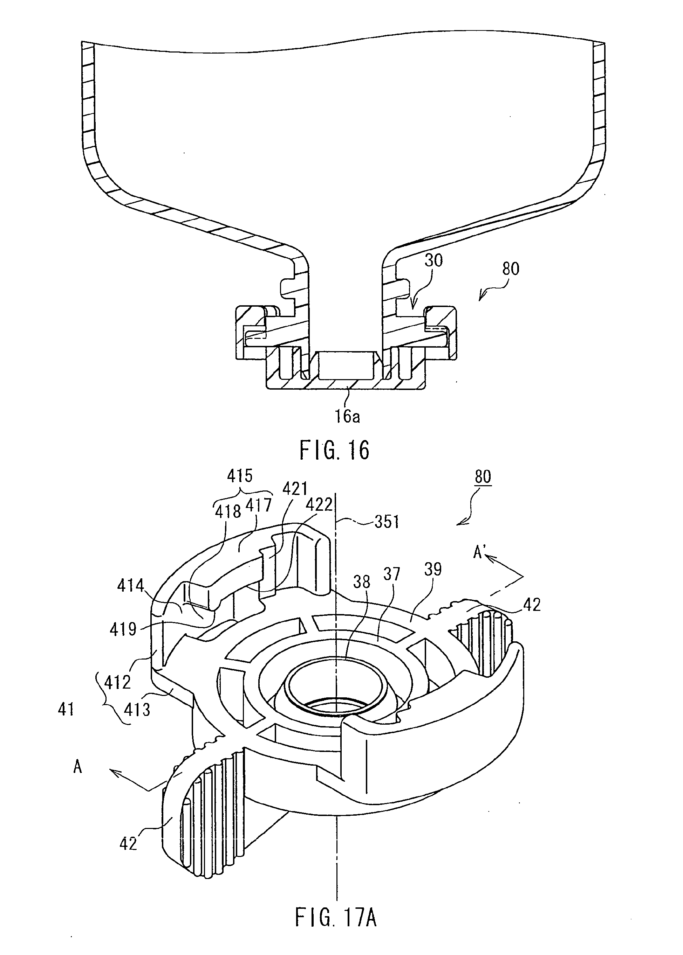

As shown in FIG. 16, an example of the male connector P (male connector 30) of the present invention described in (Embodiment 2) may be provided, for example, with a cap 80 attached thereto, when the female connector 35 is not connected to the male connector 30.

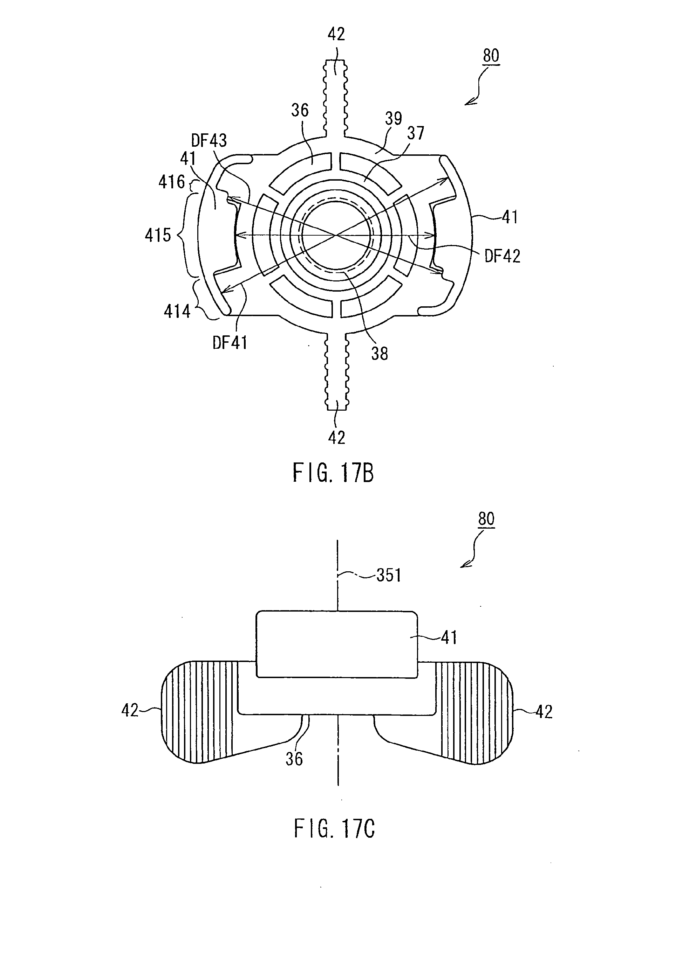

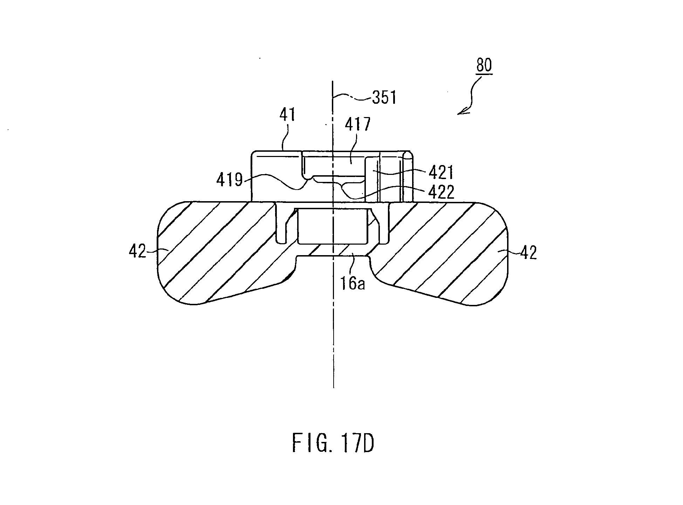

As shown in FIGS. 17A to 17D, the difference between the cap 80 and the female connector 35 described in (Embodiment 2) lies in that the cap 80 does not have the liquid-sending tube connecting section 40 (see FIG. 9E, etc.), and the cap 80 has a sealing bottom section 16a that closes an opening of the tubular section 31 when the cap 80 is attached to the male connector 30. The other configurations are the same as those of the female connector 35 described in (Embodiment 2). In FIGS. 17A to 17D, the members having the same shapes as those of the members constituting the female connector 35 described in (Embodiment 2) are denoted with the same member numbers as those of the members constituting the female connector 35, and the descriptions thereof are omitted.

### (Embodiment 7)

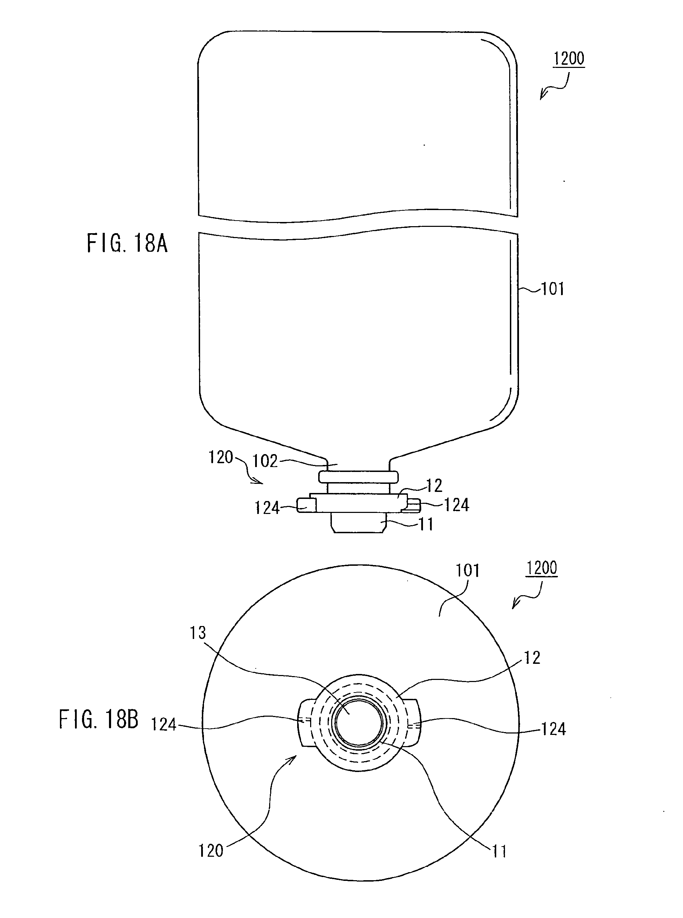

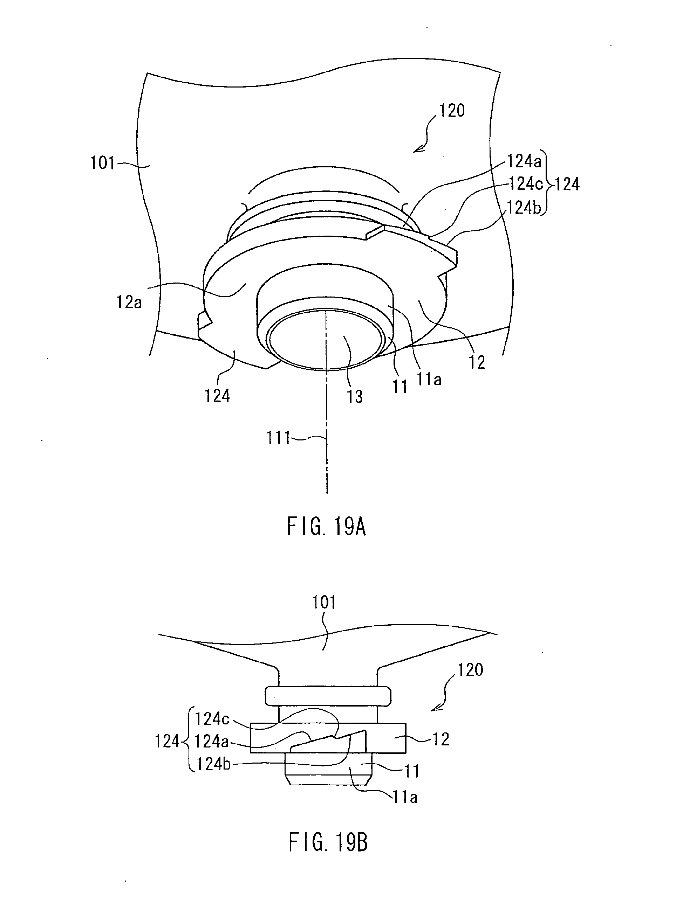

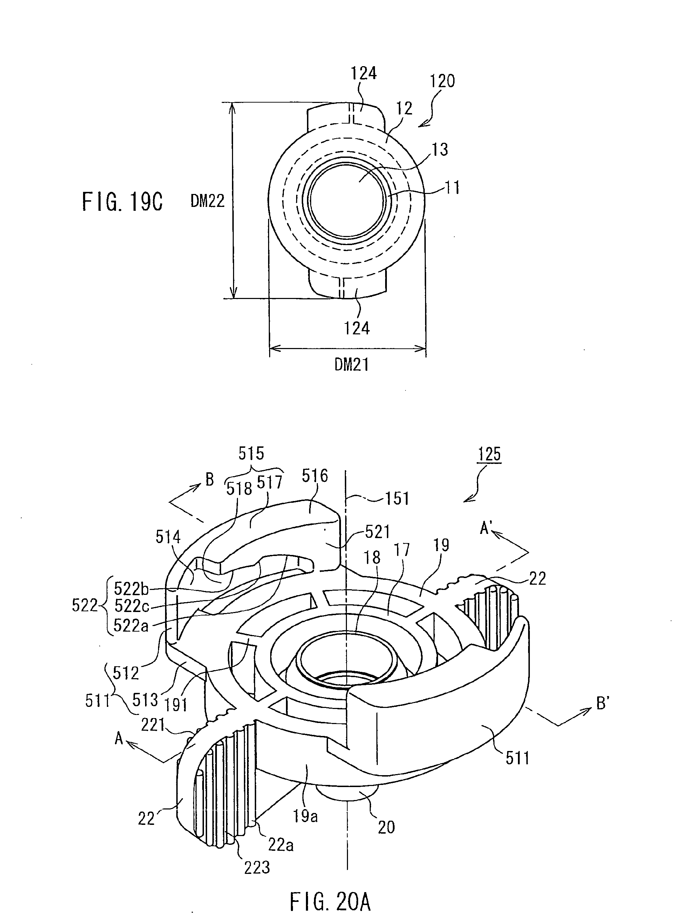

FIG. 18A is a front view showing a schematic configuration of an example of a medical container (medical container 1200) including an example of a male connector C (male connector 120) of the present invention to which an example of a female connector A (female connector 125) of the present invention is to be connected. FIG. 18B is a bottom view of the medical container shown in FIG. 18A. FIG. 19A is a partially enlarged perspective view of the medical container of FIG. 18A. FIG. 19B is a partially enlarged front view of the medical container of FIG. 18A. FIG. 19C is a partially enlarged bottom view of the medical container of FIG. 18A (it should be noted that a container main section is not shown). In FIG. 19A, alternate long and short dashed lines 111 indicate a center axis of the male connector 120. The direction of the center axis 111 is defined as an up-and-down direction, and an upper side on the drawing surface (a container main section 101 side of the medical container 1200) of FIG. 19A is referred to as an "upper side" and a lower side on the drawing surface (side to which the female connector 125 is to be connected) is referred to as a "lower side".

As shown in FIGS. 18A to 19C, the male connector of the present embodiment has the same configuration as that of the male connector described in (Embodiment 1) except that the shape of an engagement tab 124 is different, and the same members are denoted with the same member numbers and the descriptions thereof are omitted.

As shown in FIGS. 19A and 19B, a surface of each engagement tab 124 on the container main section 101 side has a stepped structure including a fourth inclined surface 124a, a third inclined surface 124b, and a second stepped surface 124c extending between the fourth inclined surface 124a and the third inclined surface 124b. The height of the fourth inclined surface 124a increases from one of both ends of the engagement tab 124 in a longitudinal direction (same direction as the circumferential direction of the tubular section 11) of the surface of the engagement tab 124 on the container main section 101 side to the center of the above-mentioned surface. The height of the third inclined surface 124b decreases from the other of both the ends of the engagement tab 124 in the longitudinal direction (same direction as the circumferential direction of the tubular section 11) of the surface of the engagement tab 124 on the container main section 101 side to the center of the above-mentioned surface.

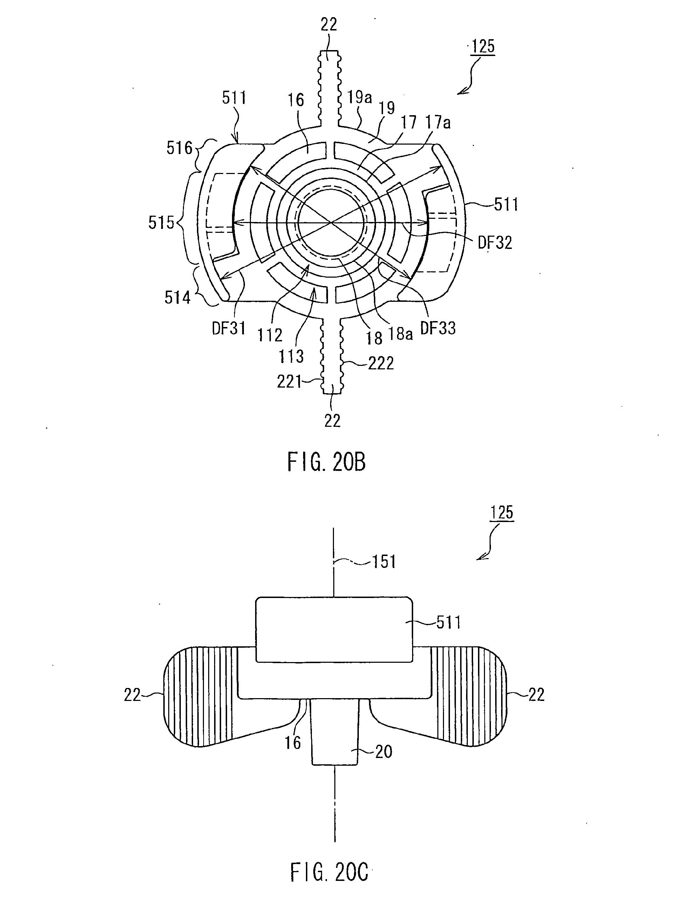

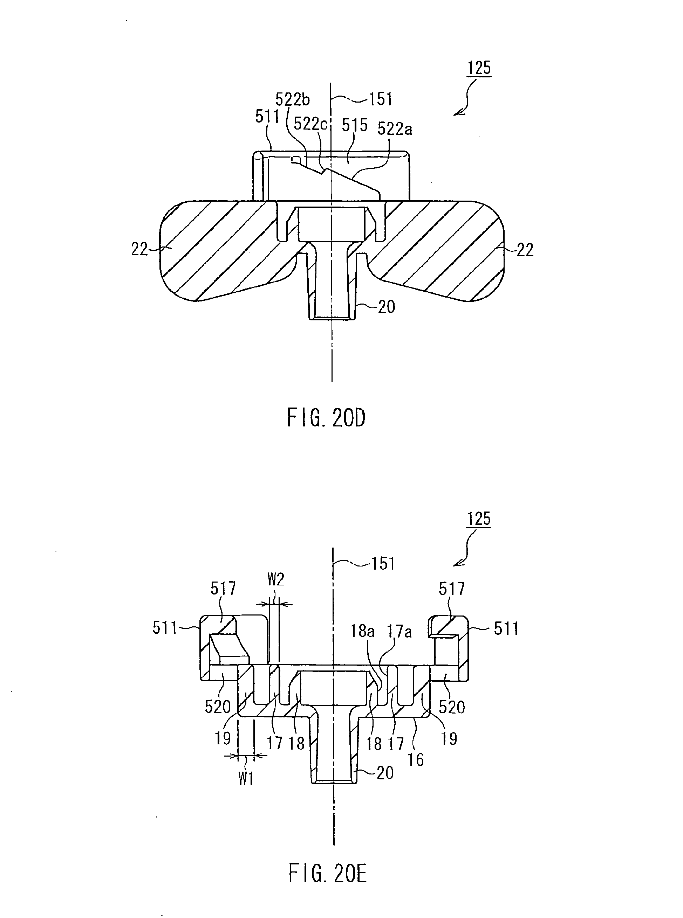

FIG. 20A is a perspective view showing a schematic configuration of an example of the female connector 125 to be connected to the male connector 120. FIG. 20B is a plan view of the female connector 125 shown in FIG. 20A FIG. 20C is a front view of the female connector 125 shown in FIG. 20A. FIG. 20D is a cross-sectional view taken along a line A-A' of the female connector 125 shown in FIG. 20A. FIG. 20E is a cross-sectional view taken along a line B-B' of the female connector 125 shown in FIG. 20A.

The female connector 125 has the same configuration as that of the female connector 15 described in (Embodiment 1), except that the shape of a brim section 511 including an arc-shaped wall 512 and a bridge section 513 is different. The same members are denoted with the same member numbers, and the descriptions thereof are omitted.

The arc-shaped wall 512 of the female connector 125 includes a passage region 514, an engagement region 515, and a non-passage region 516 (see FIG. 20B) in accordance with the difference in shape of the inner circumferential surface opposed to the center axis 151. The inner circumferential surface of the passage region 514 is a part of a cylindrical surface with a diameter DF31, and the inner circumferential surface of a non-passage region 516 is a part of a cylindrical surface with a diameter DF33 (DF33 < DF31). The engagement region 515 between the passage region 514 and the non-passage region 516 includes an engagement wall 517 at an upper end, which extends in the circumferential direction so as to connect the upper end of the passage region 514 to the upper end of the non-passage region 516. The inner circumferential surface of the engagement wall 517 opposed to the center axis 151 is a part of a cylindrical surface with a diameter DF32 (DF32 < DF31, DF33 = DF32). Due to the presence of the engagement wall 517 in the engagement region 515, a region between the engagement wall 517 and the bridge section 513 is dented in a concave shape. More specifically, the region between the engagement wall 517 and the bridge section 513 in the engagement region 515 is recessed relatively from the engagement wall 517 with respect to the center axis 151 to form a storage section 518 capable of storing the engagement tab 124 (see FIG. 19B, etc.). The non-passage region 516 adjacent to the engagement region 515 in the circumferential direction includes a convex portion 521 that protrudes relative to the engagement wall 517 in the engagement region 515 with respect to the center axis 151. In a portion of the bridge section 513, facing the engagement wall 517, a through-hole 520 (see FIG. 20E) passing through the bridge section 513 in a thickness direction thereof is formed; however, the through-hole 520 need not be provided

A surface 522 of the engagement wall 517 facing the bridge section 513 (surface by which the engagement wall 515 faces the engagement tab 124 when the male connector 120 and the female connector 125 are engaged with each other) has a stepped structure including a first inclined surface 522b, a second inclined surface 522a, and a first stepped surface 522c extending between the first inclined surface 522b and the second inclined surface 522a. The surface 522 includes the first inclined surface 522b, the first stepped surface 522c, and the second inclined surface 522a in this order from the passage region 214side. While the female connector 125 is connected to the male connector 120, the first inclined surface 522b comes into contact with the third inclined surface 124b of the engagement tab 124, the second inclined surface 522a comes into contact with the fourth inclined surface 124a of the engagement tab 124, and the first stepped surface 522c comes into contact with the second stepped surface 124c of the engagement tab 124. Thus, in the case where the engagement tab 124 of the male connector 120 has a parallel plane (fourth inclined surface 124a) that slides over an inclined surface (first inclined surface 522b) during the connection of the female connector 125 with the male connector 120, when the engagement tab 124 mounts the inclined surface (first inclined surface 522b) in the sliding direction of the engagement tab 124 with respect to the engagement wall 517 during the connection between the female connector 125 and the male connector 120, the degradation of a portion of the engagement tab 124, which comes into contact with the inclined surface (first inclined surface 522b), involved in the sliding, is suppressed.) Further, since the contact area between the engagement wall 517 and the engagement tab 124 is larger than, for example, that of the first lock protrusion 14b of the engagement tab 14 of the male connector 10 described in (Embodiment 1), a stress acting in a direction opposite to the rotation direction of the female connector 125 during the engagement is dispersed, which can prevent the damage and the like of the engagement wall 517 and/or the engagement tab 124 involved in the concentration of the stress.

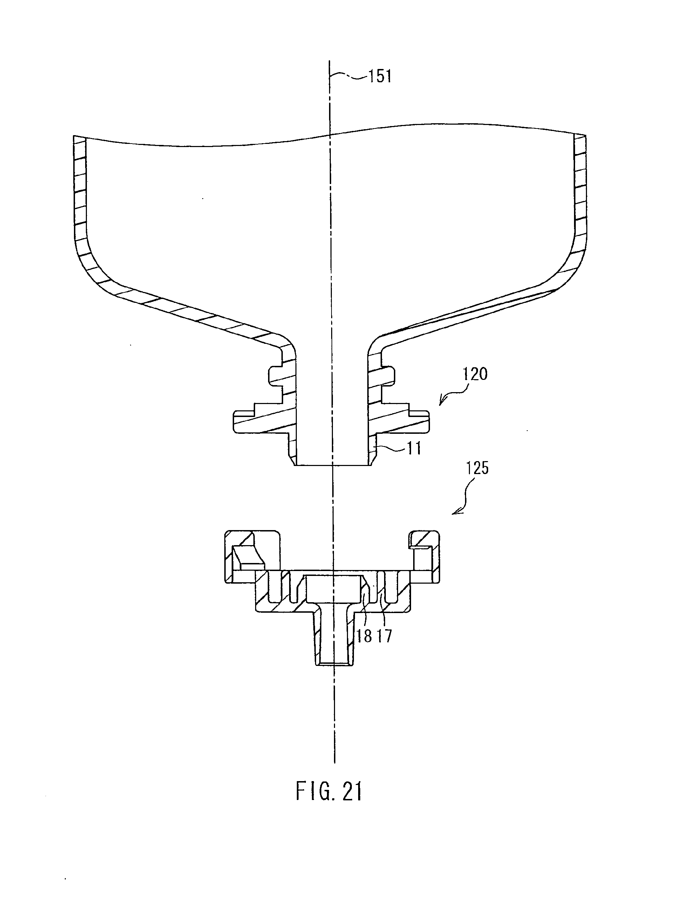

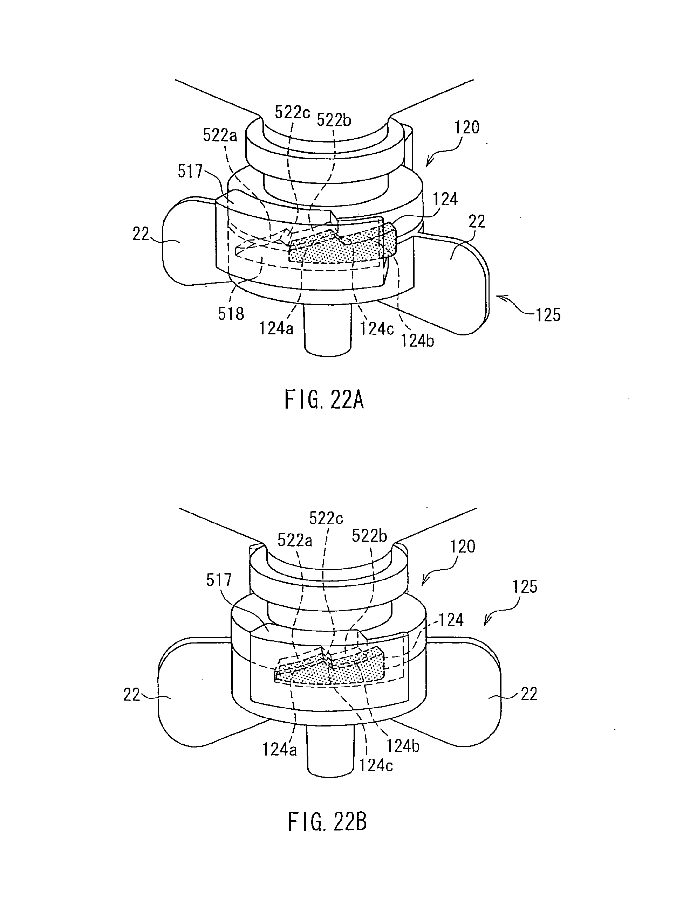

Next, a method for connecting the female connector 125 to the male connector 120 will be described. As shown in FIG. 21, the tubular section 11 of the male connector 120 is inserted in the space between the tube-shaped section 18 and the medium-diameter tube-shaped section 17 of the female connector 125. Then, for example, when the female connector 125 is rotated slightly around the center axis in this state, the fourth inclined surface 124a of the engagement tab 124 comes into contact with the inclined surface (first inclined surface 522b) of the engagement wall 517, as shown in FIG. 22A.

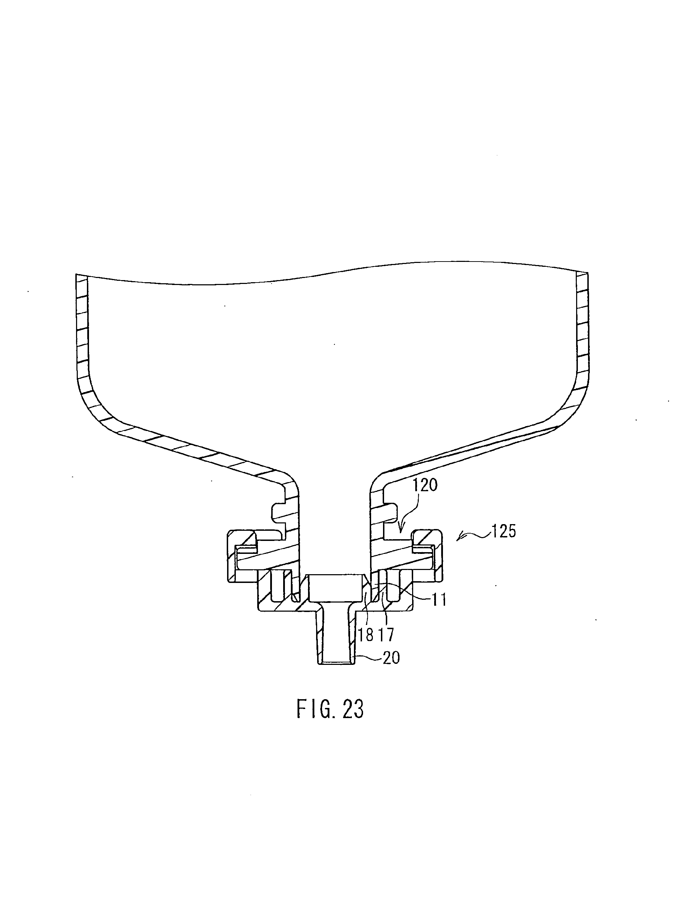

Then, the female connector 125 is rotated around the center axis 151 with respect to the male connector 120 with the grasping sections 22 grasped. As shown in FIGS. 22A and 22B, the fourth inclined surface 124a slides over the first inclined surface 522b while being pressed against the first inclined surface 522b along with the rotation of the female connector 125, and the engagement tab 124 mounts the first inclined surface 522b in the sliding direction of the engagement tab 124 with respect to the engagement wall 517. Therefore, a force acting so that the tubular section 11 of the male connector 120 is pushed in between the tube-shaped section 18 and the medium-diameter tube-shaped section 17 of the female connector 125 is provided to the male connector 120 and the female connector 125, along with the rotation of the female connector 125. Therefore, the engagement tab 124 is stored in the storage section 518, and the tube-shaped section 11 is inserted more deeply between the tube-shaped section 18 and the medium-diameter tube-shaped section 17, whereby a state shown in FIG. 23 is obtained. When the fourth inclined surface 124a passes the top of the first inclined surface 522b, the engagement tab 124 moves slightly to the upper side on the drawing surface, and the fourth inclined surface 124a comes into contact with the second inclined surface 522a. When the fourth inclined surface 124a moves over the first stepped surface 522c, the connection between the female connector 125 and the male connector 120 is not cancelled easily

Thus, even when both the male connector 120 and the female connector 125 are made of a material having a hardness higher than that of a material having flexibility and elasticity such as natural rubber, or synthetic rubber, the tube-shaped section 18 can be inserted easily in a deeper portion of the tubular section 11, and high liquid-tightness also can be ensured. Further, the sliding resistance of the engagement tab 124 with respect to the first inclined surface 522b also can be decreased. Further, since the female connector 125 is made of a material having a hardness higher than that of a material having flexibility and elasticity such as natural rubber, or synthetic rubber, a liquid-sending tube having flexibility can be connected easily to the liquid-sending tube connecting section 20 of the female connector 125, and in particular, the liquid-sending tube can be connected easily to the liquid-sending tube connecting section by a machine. Accordingly, the cost also can be reduced.

The female connector 125 is provided with the convex portion 521 (see FIG. 20A) adjacent to the storage section 518 in which the engagement tab 124 is to be stored. Therefore, if the engagement tab 124 of the male connector 120 is allowed to enter the storage section 518 and move further until coming into contact with the convex portion 521, the engagement tab 124 and the engagement wall 517 can be engaged with each other reliably. Thus, the engagement tab 124 and the engagement wall 517 can be engaged with each other reliably by a simple operation of rotating the female connector 125 with respect to the male connector 120 until the rotation is regulated.

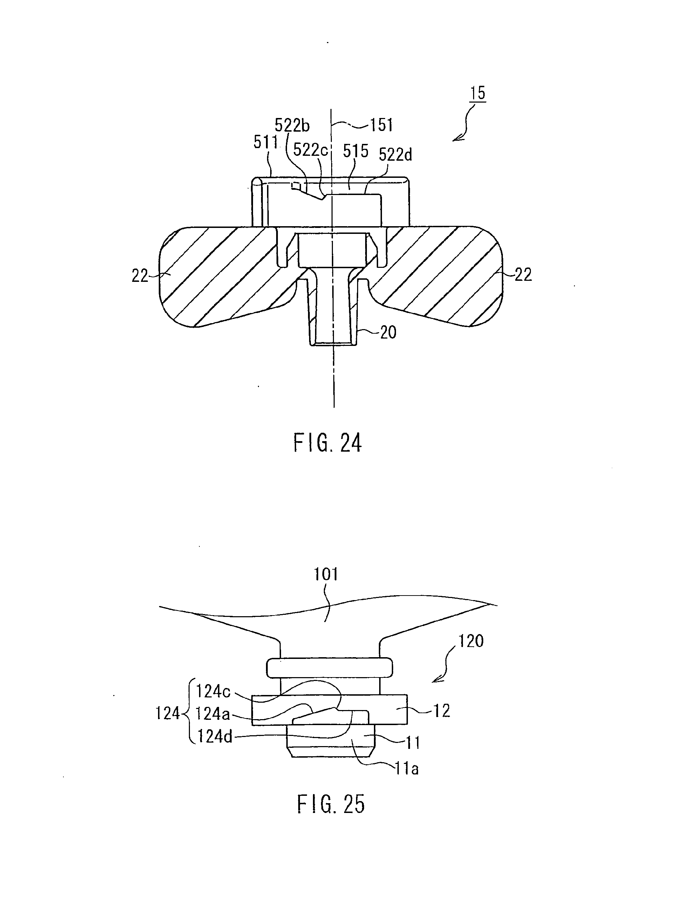

As shown in FIG. 24, a surface of the engagement wall of the female connector of the present embodiment on the bridge section side may include a horizontal surface 522d extending to the opposite side of the first stepped surface 522c on the first inclined surface 522b side, in place of the second inclined surface 522a. Further, as shown in FIG. 25, a surface of the engagement tab 124 of the male connector 120 opposite to a surface thereof on the tubular section side may include a horizontal surface 124d extending to a side of the second stepped surface 124c opposite to the fourth inclined surface 124a side, in place of the third inclined surface.

Regarding the female connector 125 and the male connector 120 described using FIGS. 22A and 22B, etc., while the female connector 125 is connected to the male connector 120, the first inclined surface 522b is in contact with the third inclined surface 124b of the engagement tab 124, the second inclined surface 522a is in contact with the fourth inclined surface 124a of the engagement tab 124, and the first stepped surface 522c is in contact with the second stepped surface 124c of the engagement tab 124, respectively However, the present embodiment also includes, for example, the case where the second stepped surface 124c and the first stepped surface 522c are not in contact with each other while the female connector 125 is connected to the male connector 120, due to the difference in inclined angle between the second stepped surface 124c and the first stepped surface 522c. Further, the present embodiment also includes, for example, the case where the first inclined surface 522b and the third inclined surface 124b are not in contact with each other, or the case where the second inclined surface 522a and the fourth inclined surface 124a are not in contact with each other, while the female connector 125 is connected to the male connector 120, due to the difference in step height between the second stepped surface 124c and the first stepped surface 522c.

### (Embodiment 8)

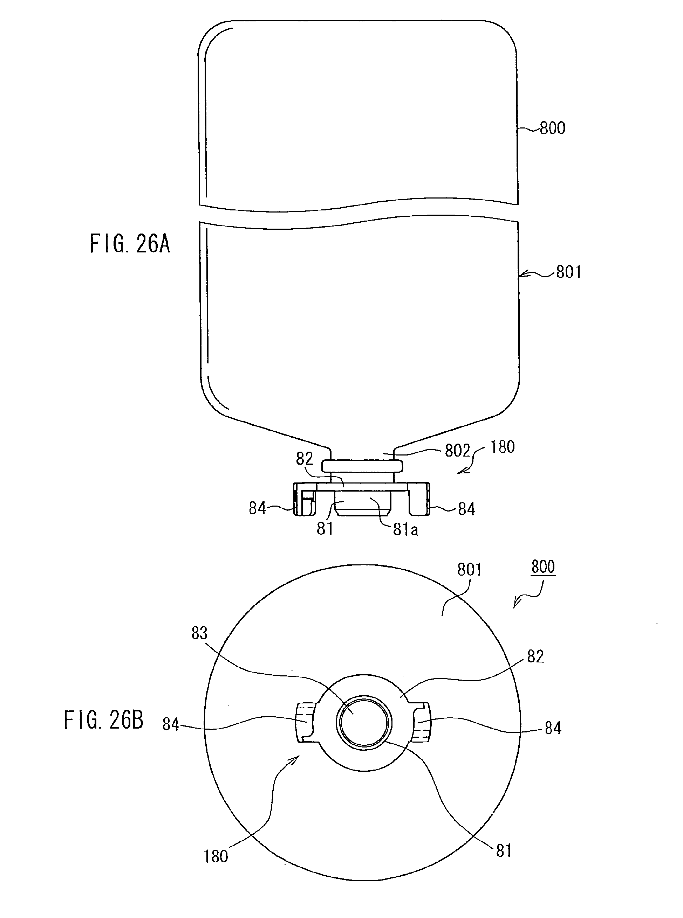

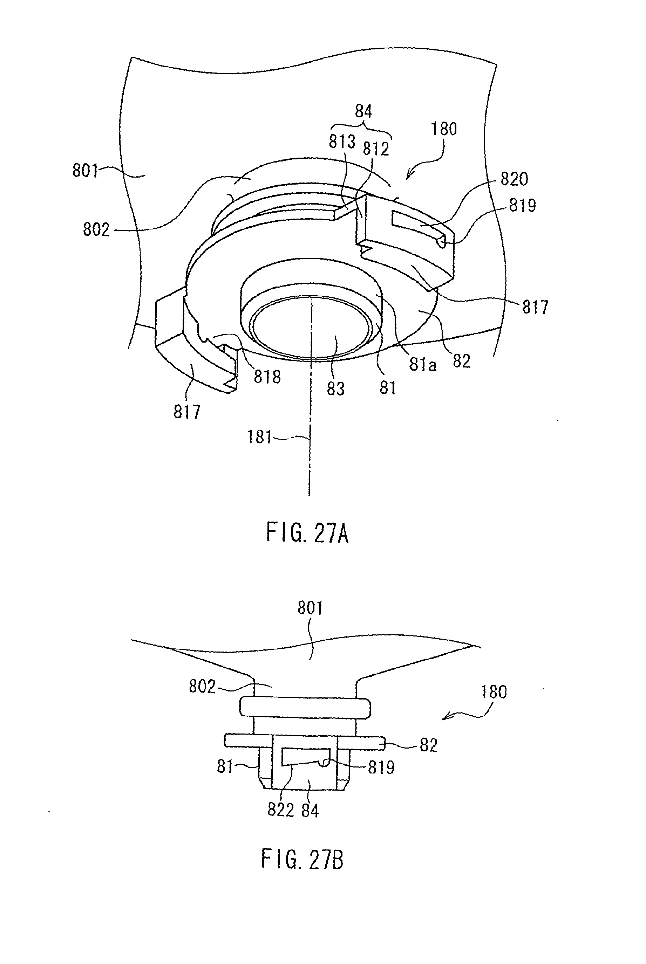

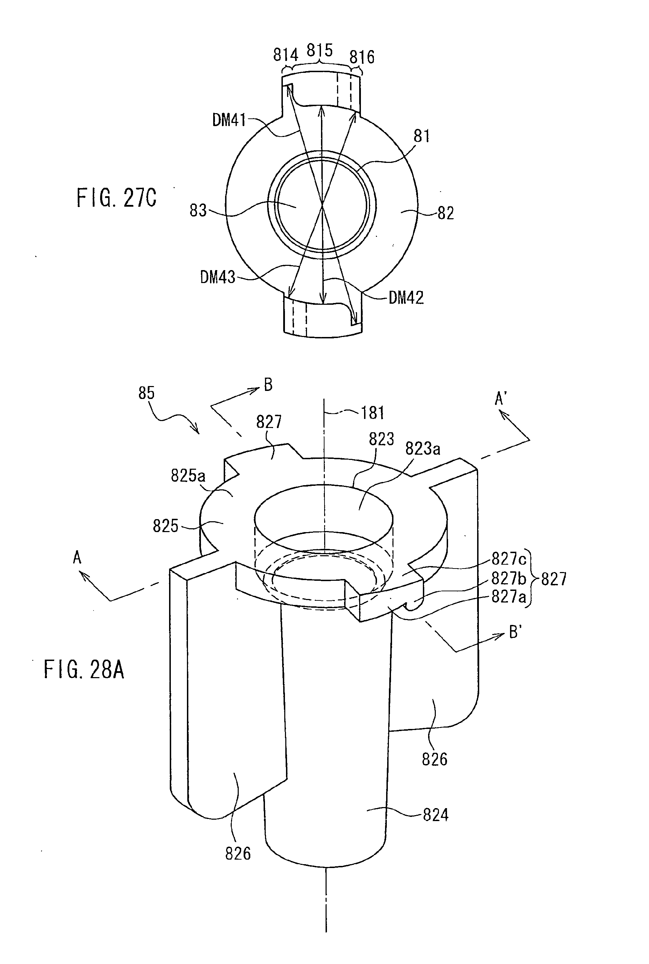

FIG. 26A is a front view showing a schematic configuration of an example of a medical container (medical container 800) including an example of a male connector E (male connector 180) of the present invention to which an example of a female connector D (female connector 85) is to be connected. FIG. 26B is a bottom view of the medical container shown in FIG.26A. FIG. 27A is a partially enlarged perspective view of the medical container of FIG. 26A. FIG. 27B is a partially enlarged front view of the medical container of FIG. 26A. FIG. 27C is a partially enlarged bottom view of the medical container of FIG. 26A (it should be noted that a container main section is not shown). In FIG. 27A, alternate long and short dashed lines 181 indicate a center axis of the male connector 180. The direction of the center axis 181 is defined as an up-and-down direction, and an upper side on the drawing surface (a container main section 801 side of the medical container 800) of FIG. 27A is referred to as an "upper side" and a lower side on the drawing surface (side to which the female connector 85 is to be connected) is referred to as a "lower side".

The medical container 800 includes the container main section 801 filled with a liquid-like substance and having a hollow cylindrical port 802 for injecting the liquid-like substance into the container main section 801 or taking out the liquid-like substance therefrom, and the male connector 180. In the example shown in FIG. 26A, the male connector 180 is integrated with the port 802 during molding. The material and hardness of the male connector 180 may be the same as those of the medical container 100 described in (Embodiment 1).

The male connector 180 has a platform 82. The platform 82 is provided with a tubular section 81 protruding downward from the center of one principal surface of the platform 82. In the tubular section 81 and the platform 82, a through-hole 83 through which the liquid-like substance in the container main section 801 flows out is formed. The platform 82 has a pair of brim sections 84, which protrude from one principal surface of the platform 82 to the tubular section 81 side and are placed around the tubular section 81. The pair of brim sections 84 are formed, for example, at symmetrical positions with respect to the center axis 181.

As shown in FIG. 27A, the brim section 84 has an arc-shaped wall 812 substantially along a cylindrical surface with the center axis 181 being the center axis, and a bridge section 813 that extends in a direction perpendicular to the center axis 181 to connect the lower end of the arc-shaped wall 812 to the platform 82.

As shown in FIG. 27C, the arc-shaped wall 812 includes a passage region 814, an engagement region 815, and a non-passage region 816 in accordance with the difference in shape of the inner circumferential surface opposed to the center axis. The inner circumferential surface of the passage region 814 is a part of a cylindrical surface with a diameter DM41, and the inner circumferential surface of the non-passage region 816 is a part of a cylindrical surface with a diameter DM43 (DM43 < DM41). The engagement region 815 between the passage region 814 and the non-passage region 816 includes an engagement wall 817 (see FIG. 27A) at an upper end, which extends in the circumferential direction so as to connect the upper end of the passage region 814 to the upper end of the non-passage region 816. The inner circumferential surface of the engagement wall 817 opposed to the center axis 181 is a part of a cylindrical surface with a diameter DM42 (DM42 < DM41). Due to the presence of the engagement wall 817 in the engagement region 815, a region between the engagement wall 817 and the bridge section 813 is dented in a concave shape with respect to the center axis 181. More specifically, the region between the engagement wall 817 and the bridge section 813 is recessed relatively from the engagement wall 817 with respect to the center axis 181 to form a storage section 818 (see FIG. 27A) for storing engagement tab 827 of the female connector described later.

The non-passage region 816 adjacent to the engagement region 815 in the circumferential direction protrudes toward the center axis 181 relative to the storage section 818. On the lower surface of the engagement wall 817 on the non-passage region 816 side (an end portion on the non-passage region 816 side of a surface of the engagement wall 817 on the concave portion 818 side, an end portion on the non-passage region 816 side of a surface of the engagement wall 817 facing the bridge section 813), a third lock concave portion 819 dented in a concave shape is formed.

In a portion of the arc-shaped wall 812 facing the engagement tab 827 when the engagement tab 827 is stored in the storage section 818, a through-hole 820 (see FIG. 27A) passing through the arc-shaped wall 812 in a thickness direction is formed. In this case, it is preferred that the through-hole 820 is formed for the reason that it is easy to confirm that the engagement tab 827 is stored in the storage section 818. However, as long as the engagement tab 827 is stored in the storage section 818, the through-hole 820 need not be formed in the arc-shaped wall 812.

A portion of a surface of the engagement wall 817 on the storage section 818 side (surface facing the bridge section 813), away from an end of the non-passage region 816 on the engagement region side relative to a portion in which the third lock concave portion 819 is formed, specifically, a region of the surface of the engagement wall 817 on the storage section 818 side, extending from one end of the surface, away from the end of the non-passage region 816 on the engagement region side, to the third lock concave portion 819 includes an inclined surface 822 (see FIG. 27B). The inclined surface 822 is inclined gradually so that the distance from the bridge section 813 decreases from one end of the surface away from the end of the non-passage region 816 on the engagement region 815 side to the third lock concave portion 819.

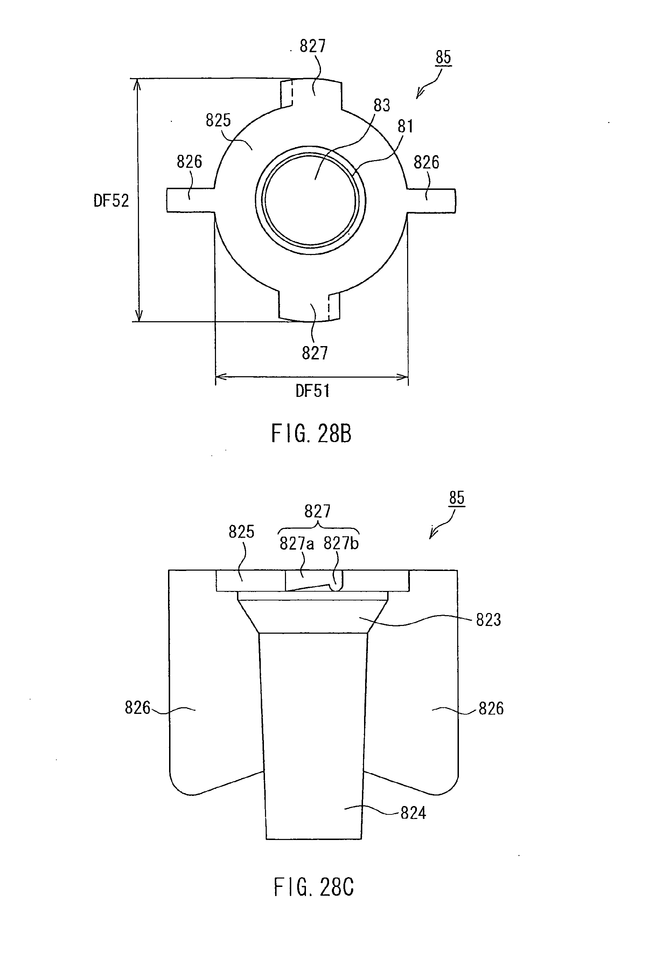

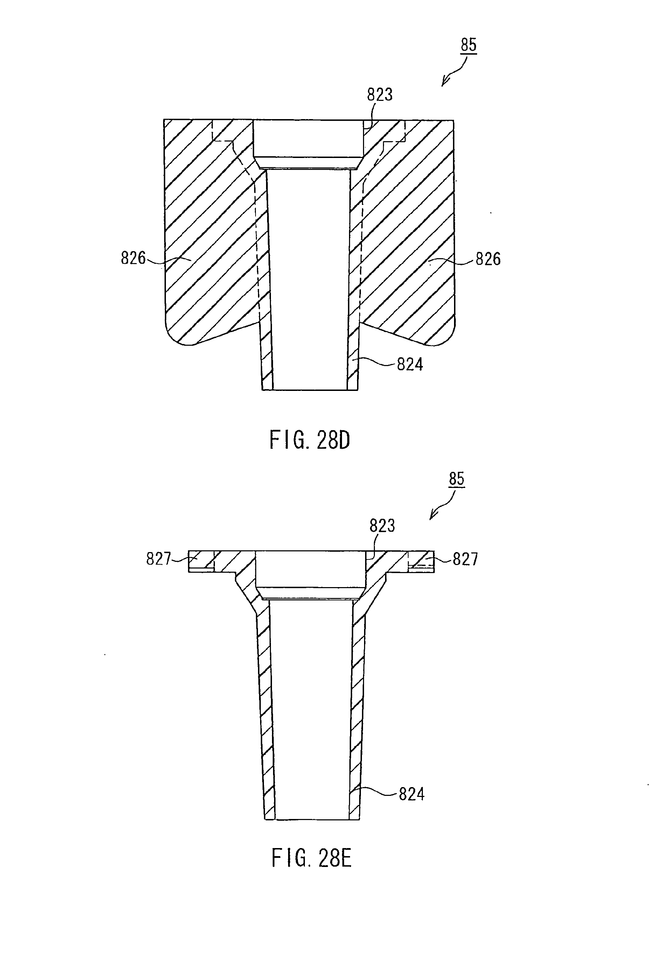

FIG. 28A is a perspective view showing a schematic configuration of an example of the female connector 85 to be connected to the male connector 180. FIG. 28B is a plan view of the female connector 85 shown in FIG. 28A. FIG. 28C is a front view of the female connector 85 shown in FIG. 28A. FIG. 28D is a cross-sectional view taken along a A-A' line of the female connector 85 shown in FIG. 28A. FIG. 28E is a cross-sectional view taken along a B-B' line of the female connector 85 shown in FIG. 28A.

As shown in FIGS. 28A to 28E, the female connector 85 includes an insertion section 823, a liquid-sending tube connecting section 824, a flange section 825, grasping sections 826, and the engagement tabs 827. Alternate long and short dashed lines 181 indicate a center axis of the insertion section 823, which is matched with the center axis of the female connector 85 and the liquid-sending tube connecting section 824. The direction of the center axis 181 is defined as an up-and-down direction, and an upper side on the drawing surface (side to be connected to the male connector 180) of FIG. 28A is referred to as an "upper side" and a lower side on the drawing surface (side to which a liquid-sending tube is to be connected) is referred to as a "lower side"

The tubular section 81 of the male connector 180 is inserted in the insertion section 823. The inner diameter of the insertion section 823 is equal to or slightly smaller than the outer diameter of the tubular section 81. Therefore, an inner circumferential surface 823a of the insertion section 823 comes into tight contact with the outer circumferential surface 81a of the tubular section 81 (see FIG. 27A, etc.) when the male connector 85 and the female connector 180 are connected to each other. Thus, the male connector 180 and the female connector 85 can be connected to each other reliably without causing the leakage of the liquid-like substance.

The insertion section 823 is connected to the tube connecting section 824. When the male connector 180 and the female connector 85 are connected to each other, an inside of the tube-connecting section 824 is communicated with the tubular section 81 of the male connector 180 and a side of the tube connecting section 824 opposite to the insertion section 823 side is connected to the liquid-sending tube.

The flange section 825 is formed so as to extend outward from an end of the insertion section 823 on the side on which the tubular section 81 is inserted, and a planar shape thereof has an annular shape. The flange section 825 is opposed to the platform 82 of the male connector 180 when the male connector 180 and the female connector 85 are connected to each other.

As shown in FIG. 28A, the engagement tabs 827 are formed so as to protrude outward from the outer circumferential surface of the flange section 825. Each engagement tab 827 includes an engagement chip 827a and a third lock protrusion 827b formed at one end of the engagement chip 827a so as to protrude downward. As shown in FIG. 28A, an upper surface 825a of the flange section 825 is a flat surface, the upper surface 825a and upper surfaces 827c of the pair of engagement tabs 827 are present on the identical plane. As shown in FIG. 28B, the outer diameter of the flange section 825 is DF51, and the distance between tops of the pair of engagement tabs 287 is DF52 (DM52 > DM51).

As long as each grasping section 826 can be grasped easily and allows rotation to be conducted easily, there is no particular limit to the shape, formation position thereof, etc.

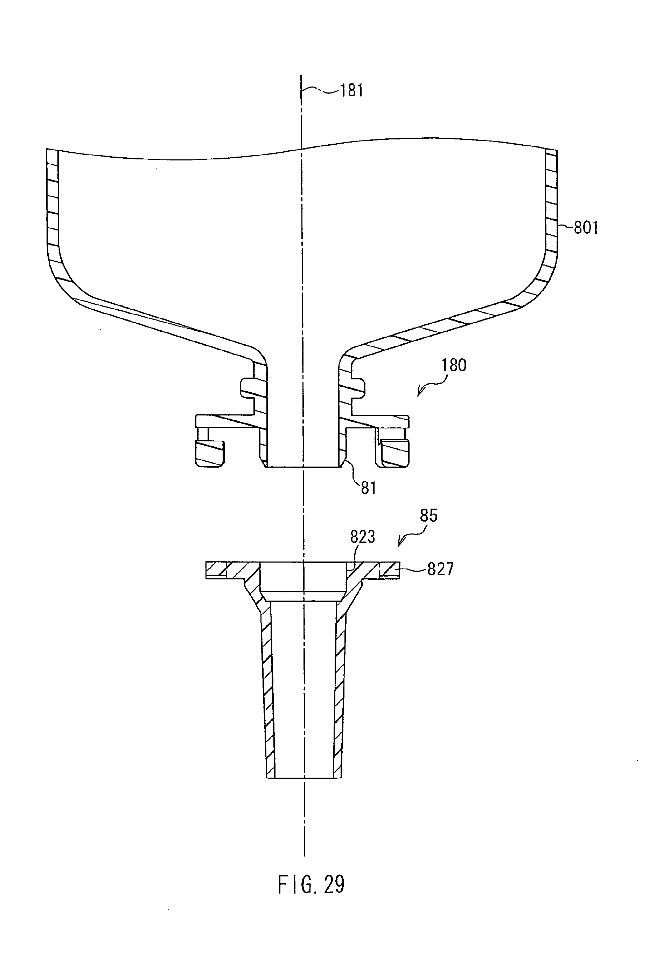

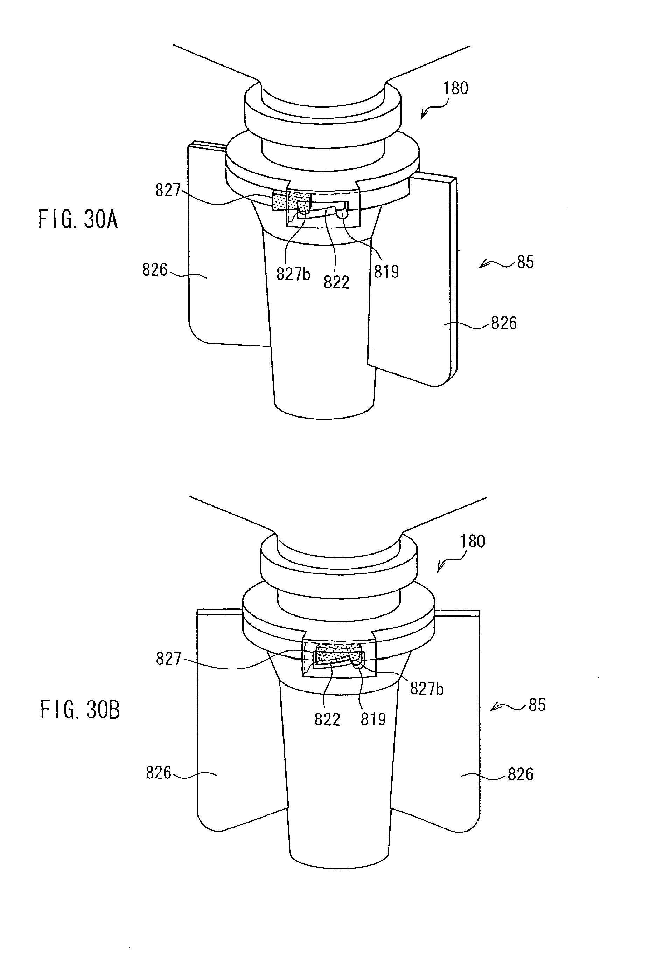

Next, a method for connecting the female connector 85 to the male connector 180 will be described. As shown in FIG. 29, the tubular section 81 of the male connector 180 is inserted in the insertion section 823 of the female connector 85. Then, for example, when the female connector 85 is rotated slightly around the center axis 181 in this state, as shown in FIG. 30A, the third lock protrusion 827b of the engagement tab 827 comes into contact with the inclined surface 822 of the engagement wall 817 (see FIG. 27A).

Then, for example, the female connector 85 is rotated around the center axis 181 with respect to the male connector 180 with the grasping sections 826 grasped. As shown in FIGS. 30A and 30B, the third lock protrusion 827b slides over the inclined surface 822 while being pressed against the inclined surface 822 along with the rotation of the female connector 85, and the engagement tab 827 mounts the inclined surface 822 in the sliding direction of the engagement tab 827 with respect to the engagement wall 817. That is, the surface of the engagement wall 817 facing the engagement tab 827 includes the inclined surface 822 which the engagement tab 827 mounts in the sliding direction of the engagement tab 827 with respect to the engagement wall 817 during the connection between the female connector 85 and the male connector 180. Therefore, for example, a force acting so that the tubular section 81 of the male connector 180 is pushed in the insertion section 823 of the female connector 85 (force at which the male connector 180 and the female connector 85 approach each other) is provided to the male connector 180 and the female connector 85, along with the rotation of the female connector 15. When the third lock protrusion 827b crosses the inclined surface 822, the third lock protrusion 827b is engaged with the third lock concave portion 819. Therefore, the engagement tab 827 is stored in the concave portion 818, and the tubular section 81 is inserted more deeply in the insertion section 823 to obtain a state shown in FIG. 30 and high liquid-tightness can also be ensured.

Thus, even when both the male connector 180 and the female connector 85 are made of a material having a hardness higher than that of a material having flexibility and elasticity such as natural rubber, or synthetic rubber, the tubular section 81 can be inserted easily in a deeper portion of the insertion section 823 and high liquid-tightness also can be ensured. Further, the sliding resistance of the engagement tab 827 with respect to the inclined surface 822 also can be decreased. Further, since the female connector 85 is made of a material having a hardness higher than that of a material having flexibility and elasticity such as natural rubber, or synthetic rubber, a liquid-sending tube having flexibility can be connected easily to the liquid-sending tube connecting section 824 of the female connector 85, and in particular, the liquid-sending tube can be connected easily to the liquid-sending tube connecting section 824 by a machine. Accordingly, a cost also can be reduced.

The male connector 180 is provided with the non-passage region 816 (see FIG. 20A) that is adjacent to the storage section 818 in which the engagement tab 827 is to be stored and that protrudes to the center axis 181 side relative to the storage section 818. Therefore, if the engagement tab 827 of the male connector 85 is allowed to enter the storage section 818 and move further until coming into contact with a surface of the non-passage region 816 facing the engagement region 815, the engagement tab 827 and the engagement wall 817 can be engaged with each other reliably. Thus, the engagement tab 827 and the engagement wall 817 can be engaged with each other reliably by a simple operation of rotating the female connector 85 with respect to the male connector 180 until the rotation is regulated.

### Industrial Applicability

The present invention can provide a male connector, a female connector, a liquid-sending circuit, a medical container, etc. that can be used preferably in an intravenous nutrition therapy as well as a transintestine nutrition therapy.

## Claims

1. A female connector to be connected to a male connector having a tubular section through which a liquid-like substance flows out,
wherein the male connector includes:
a platform in which the tubular section is provided so as to protrude from a center of one principal surface; and
an engagement tab protruding outward from an outer circumferential surface of the platform,
the female connector includes:
a tube-shaped section to be connected to the tubular section;
a bottom section connected to one end of the tube-shaped section;
a liquid-sending tube connecting section to which a liquid-sending tube is to be connected, one end of the liquid-sending tube connecting section being connected to the tube-shaped section through the bottom section of the female connector, and an inside of the liquid-sending tube connecting section being communicated with an inside of the tubular section when the female connector is connected to the male connector; and
a brim section provided so that at least a part thereof protrudes to a side of the tube-shaped section opposite to the liquid-sending tube connecting section side,
an inner circumferential surface of the brim section opposed to a center axis of the tube-shaped section includes an engagement wall protruding inward with a longitudinal direction thereof being in a circumferential direction, and a part of the inner circumferential surface of the brim section on the tube-shaped section side relative to the engagement wall, which is recessed with respect to the center axis relative to the engagement wall due to the presence of the engagement wall, constitutes a concave portion in which the engagement tab is to be stored, and
a surface of the engagement wall facing the engagement tab when the female connector is connected to the male connector includes an inclined surface that the engagement tab mounts in a sliding direction of the engagement tab with respect to the engagement wall during the connection between the female connector and the male connector.

2. The female connector according to claim 1, wherein when the female connector is connected to the male connector, an outer circumferential surface of the tube-shaped section is in contact with an inner circumferential surface of the tubular section, and
the female connector further includes a medium-diameter tube-shaped section, at least a part of an inner circumferential surface of which is in contact with an outer circumferential surface of the tubular section and one end of which is connected to the tube-shaped section through the bottom section.

3. The female connector according to claim 2, further comprising a large-diameter tube-shaped section which is placed around the medium-diameter tube-shaped section so that a space is formed between the medium-diameter tube-shaped section and the large-diameter tube-shaped section, and one end of which is connected to the medium-diameter tube-shaped section through the bottom section,
wherein a thickness of the large-diameter tube-shaped section is larger than that of the medium-diameter tube-shaped section, and the brim section is connected to the medium-diameter tube-shaped section through the large-diameter tube-shaped section.

4. The female connector according to claim 3, further comprising a cross-linking section that is placed between the medium-diameter tube-shaped section and the large-diameter tube-shaped section and partially connects the medium-diameter tube-shaped section to the large-diameter tube-shaped section.

5. The female connector according to claim 3 or 4, further comprising a grasping section that protrudes outward from an outer circumferential surface of the large-diameter tube-shaped section.

6. The female connector according to any one of claims 1 to 5, wherein the engagement tab includes an engagement chip and a first lock protrusion formed on a surface of the engagement chip opposite to a surface of the engagement chip on the tubular section side, and
a surface of the engagement wall facing the engagement tab when the female connector is connected to the male connector includes a first lock concave portion which is connected to the inclined surface and in which the first lock protrusion having slid over the inclined surface is to be stored.

7. The female connector according to any one of claims 1 to 5, wherein when the inclined surface is defined as a first inclined surface,
a surface of the engagement wall facing the engagement tab has a stepped structure including the first inclined surface, a second inclined surface, and a first stepped surface extending between the first inclined surface and the second inclined surface, and
when the female connector is connected to the male connector, the engagement tab includes a third inclined surface opposed to the first inclined surface, a fourth inclined surface opposed to the second inclined surface, and a second stepped surface opposed to the first stepped surface.

8. A male connector to be connected to the female connector according to claim 7, comprising:
a platform in which the tubular section is provided so as to protrude from a center of one principal surface; and
an engagement tab protruding outward from an outer circumferential surface of the platform,
wherein the engagement tab includes a third inclined surface opposed to the first inclined surface, a fourth inclined surface opposed to the second inclined surface, and a second stepped surface opposed to the first stepped surface, when the female connector is connected to the male connector.

9. A male connector to be connected to a female connector, having a tubular section through which a liquid-like substance flows out,
wherein the male connector includes:
a platform in which the tubular section is provided so as to protrude from a center of one principal surface; and
an engagement tab protruding outward from an outer circumferential surface of the platform,
the female connector includes:
a tube-shaped section to be connected to the tubular section;
a bottom section connected to one end of the tube-shaped section;
a liquid-sending tube connecting section to which a liquid-sending tube is to be connected, one end of the liquid-sending tube connecting section being connected to the tube-shaped section through the bottom section of the female connector, and an inside of the liquid-sending tube connecting section being communicated with an inside of the tubular section when the female connector is connected to the male connector; and
a brim section provided so that at least a part thereof protrudes to a side of the tube-shaped section opposite to the liquid-sending tube connecting section side,
an inner circumferential surface of the brim section opposed to a center axis of the tube-shaped section includes an engagement wall protruding inward with a longitudinal direction thereof being in a circumferential direction, and a part of the inner circumferential surface of the brim section on the tube-shaped section side relative to the engagement wall, which is recessed with respect to the center axis relative to the engagement wall due to the presence of the engagement wall, constitutes a concave portion in which the engagement tab is to be stored, and
a surface of the engagement tab facing the engagement wall when the female connector is connected to the male connector includes an inclined surface that the engagement wall mounts in a sliding direction of the engagement wall with respect to the engagement tab during the connection between the female connector and the male connector.

10. The male connector according to claim 9, wherein a surface of the engagement wall facing the engagement tab when the female connector is connected to the male connector includes a second lock protrusion, and
the engagement tab includes a second lock concave portion in which the second lock protrusion having slid over the inclined surface is to be stored.

11. The male connector according to claim 9 or 10, wherein when the male connector is not connected to the female connector, an opening of the tubular section is closed with a cap.

12. The male connector according to claim 10, wherein the cap includes members having the same structures as those of the tube-shaped section, the bottom section, and the brim section of the female connector, and sealing bottom section that closes the opening of the tubular section of the male connector when the cap is attached to the male connector, in place of the liquid-sending tube connecting section of the female connector.

13. A connection structure of the female connector according to any one of claims 1 to 7 and a male connector having a tubular section through which a liquid-like substance flows out,
wherein the male connector includes:
a platform in which the tubular section is provided so as to protrude from a center of one principal surface; and
an engagement tab protruding outward from an outer circumferential surface of the platform, and
the engagement tab and the engagement wall of the female connector are engaged with each other when the engagement tab is stored in the concave portion of the female connector.

14. A connection structure of the male connector according to any one of claims 9 to 12 and a female connector,
wherein the female connector includes:
a tube-shaped section to be connected to the tubular section;
a bottom section connected to one end of the tube-shaped section;
a liquid-sending tube connecting section to which a liquid-sending tube is to be connected, one end of the liquid-sending tube connecting section being connected to the tube-shaped section through the bottom section of the female connector, and an inside of the liquid-sending tube connecting section being communicated with an inside of the tubular section when the female connector is connected to the male connector; and
a brim section provided so that at least a part thereof protrudes to a side of the tube-shaped section opposite to the liquid-sending tube connecting section side,
an inner circumferential surface of the brim section opposed to a center axis of the tube-shaped section includes an engagement wall protruding inward with a longitudinal direction thereof being in a circumferential direction, and a part of the inner circumferential surface of the brim section on the tube-shaped section side relative to the engagement wall, which is recessed with respect to the center axis relative to the engagement wall due to the presence of the engagement wall, constitutes a concave portion in which the engagement tab is to be stored, and
the engagement tab and the engagement wall are engaged with each other when the engagement tab of the male connector is stored in the concave portion.

15. A male connector to be connected to a female connector, having a tubular section through which a liquid-like substance flows out,
wherein the male connector includes:
a platform in which the tubular section is provided so as to protrude from a center of one principal surface; and
a brim section that protrudes from the one principal surface of the platform to the tubular section side and is placed around the tubular section,
the female connector includes:
an insertion section in which the tubular section is to be inserted;
a liquid-sending tube connecting section to which a liquid-sending tube is to be connected, the liquid-sending tube connecting section being connected to the insertion section and an inside of the liquid-sending tube connecting section being communicated with an inside of the tubular section when the female connector is connected to the male connector;
a flange section opposed to the platform when the female connector is connected to the male connector; and
an engagement tab protruding outward from an outer circumferential surface of the flange section,
an inner circumferential surface of the brim section opposed to a center axis of the tubular section of the male connector includes an engagement wall protruding inward with a longitudinal direction thereof being in a circumferential direction, and a part of the inner circumferential surface of the brim section on the platform side relative to the engagement wall, which is recessed with respect to the center axis relative to the engagement wall due to the presence of the engagement wall, constitutes a storage section in which the engagement tab is to be stored, and
a surface of the engagement wall facing the engagement tab when the female connector is connected to the male connector includes an inclined surface that the engagement tab mounts in a sliding direction of the engagement tab with respect to the engagement wall during the connection between the female connector and the male connector.

16. A liquid-sending circuit, comprising: the female connector according to any one of claims 1 to 7; and a liquid-sending tube connected to the liquid-sending tube connecting section of the female connector.

17. A medical container, comprising:
a container main section having a hollow cylindrical port for injecting a liquid-like substance into the container main section or taking out the liquid-like substance therefrom; and
the male connector according to any one of claims 8 to 12,
wherein the male connector is integrated with the port in the course of molding or connected to the port removably through connection means.
